# EUROPEAN PATENT SPECIFICATION

(11) **EP 4 076 372 B1**
(45) Date of publication and mention of the grant of the patent: **11.11.2026**
(21) Application number: 20838047.7
(22) Date of filing: 18.12.2020
(51) Int. Cl.: A61K 8/73, A61K 8/81, A61Q 5/06

(54) **COMPOSITION COMPRISING A DISPERSION OF POLYMER PARTICLES IN A NON-AQUEOUS MEDIUM, A CATIONIC POLYMER AND AN ANIONIC POLYMER**
ZUSAMMENSETZUNG MIT EINER DISPERSION VON POLYMERPARTIKELN IN EINEM NICHT WÄSSRIGEN MITTEL, EINEM KATIONISCHEN POLYMER UND EINEM ANIONISCHEN POLYMER
COMPOSITION COSMÉTIQUE COMPRENANT UNE DISPERSION DE PARTICULES DE POLYMÈRES DANS UN MILIEU NON AQUEUX, UN POLYMÈRE CATIONIQUE ET UN POLYMÈRE ANIONIQUE.

(30) Priority: 20.12.2019 FR 1915116
(43) Date of publication of application: 26.10.2022
(73) Proprietor: L'OREAL, 75008 Paris (FR)
(72) Inventor: RONCHARD, Guillaume, 93400 SAINT-OUEN (FR); SOULIE, Virginie, 93400 Saint-Ouen (FR); RIVIERE, Mathilde, 93400 Saint-Ouen (FR); VOISIN, Sébastien, 93400 SAINT-OUEN (FR)
(74) Representative: L'Oreal
(86) International application number: PCT/EP2020/087224
(87) International publication number: WO 2021/123309

(56) References cited:
- EP-A1- 0 853 470
- WO-A1-03/066010
- WO-A1-2018/230673
- FR-A1- 3 075 628

## Description

The present invention relates to a composition, preferably an aqueous composition, comprising at least one dispersion of polymer particles stabilized in a non-aqueous medium, at least one cationic polymer and at least one anionic polymer.

The present invention also relates to a cosmetic treatment process, notably a process for styling keratin fibres, in particular human keratin fibres such as the hair, using this cosmetic composition.

Finally, the present invention relates to the use of this composition for the cosmetic treatment of keratin fibres, in particular of human keratin fibres such as the hair, and in particular for styling the hair, i.e. for shaping and/or fixing the head of hair.

Styling products are normally used to construct and structure the hairstyle and to give it hold. They are usually in the form of lotions, gels, foams, creams or sprays. These compositions generally comprise one or more film-forming polymers or "fixing polymers", enabling the formation of a coating film on the hair strands and thus ensuring hold of the hairstyle and/or the formation of micro-welds between the hair strands, thus ensuring the fixing of the head of hair.

These compositions are generally applied to wet hair, which is shaped before performing blow-drying or drying.

To obtain satisfactory and long-lasting fixing power, it is known practice to incorporate into styling products polymers with very high fixing power, and/or to increase the concentration of fixing polymer. However, the use of such extremely fixing products causes a certain number of drawbacks.

Although the objective of these products is to ensure the fixing and the hold of the hairstyle over time, they generally have a tendency to make the hairstyle rigid, notably producing a "helmet effect", which is often poorly perceived by users.

The head of hair, thus made rigid, has a dry and rough feel which is not greatly appreciated by consumers.

Moreover, when the styling products are in the form of mousses, they may have more or less firm textures with limited hold in the hand. Conversely, the textures of gels may be greasy or tacky and may prove difficult to apply to the head of hair. Compositions that do not leave any residues on the hair are also sought. FR 3 075 628 A1 (L'OREAL) discloses a composition comprising: (a) a or more dispersions of particles of at least one polymer, stabilized by one or more stabilizing agents in a non-aqueous medium, (b) one or more hydrophobic film-forming polymers, preferably chosen from hydrocarbon block copolymers, hydrocarbon resins and mixtures thereof, and (c) water. The polymer (a) is preferably chosen from methyl acrylate / ethyl acrylate / acrylic acid / isobornyl acrylate (10/60/20/10) copolymer (examples A1-A6).

Thus, there is a real need to develop styling compositions which do not have the drawbacks mentioned above, i.e. which afford long-lasting fixing of the hairstyle, with styling effects that persist throughout the day or even for several days, while at the same time maintaining a natural and non-rigid appearance for the hairstyle.

There is in particular a need to develop compositions which give good styling properties, notably in terms of flexibility and volume, while at the same time affording a pleasant cosmetic feel, notably a soft, smooth feel, and a clean look, which is neither mattifying nor too shiny. It is also expected that the hair roots should be lifted, so that the head of hair can gain volume.

There is also a need to develop compositions with a non-tacky and non-greasy texture, which are easy to apply.

The aim of the present invention is also to provide a composition which can afford good hair fixing and hold properties, while at the same time conserving a clean and non-tacky feel of the hair, free of residues, and which may optionally be used directly during showering or shampooing.

The Applicant has discovered, surprisingly, that the combination of a dispersion of polymer particles, stabilized in a non-aqueous medium, of a cationic polymer and of an anionic polymer makes it possible to achieve the objectives outlined above, notably to obtain a styling composition that is easy to apply and that is capable of giving the hairstyle long-lasting fixing while at the same time conserving a natural, non-rigid appearance, and also a soft, smooth feel.

The subject of the present invnetion is disclosed in teh appended claims and relates to a composition comprising:
(a) one or more dispersions of particles of at least one polymer, stabilized with one or more polymer stabilizers in a non-aqueous medium,
(b) one or more cationic polymers, and
(c) one or more anionic polymers, wherein:
   - (a1) the stabilizer is an isobornyl (meth)acrylate polymer,
   - (a2) the polymer (a) in the form of particles is an ethylenic polymer,
   - (b1) the cationic polymers are chosen from homopolymers or copolymers including one or more units derived from vinylamine and optionally one or more units derived from vinylformamide, cationic polysaccharides, alkyldiallylamine or dialkyldiallylammonium cyclopolymers, and their mixtures,
   - (c1) the anionic polymers are chosen from crosslinked or non-crosslinked acrylic or methacrylic acid homopolymers or copolymers and salts thereof, crosslinked or non-crosslinked 2-acrylamido-2-methylpropanesulfonic acid homopolymers and salts thereof and crosslinked or non-crosslinked acrylamide copolymers thereof and salts thereof, the branched block butyl acrylate/ acrylic acid/methacrylic acid anionic polymer (INCI name: AMP-acrylates/allyl methacrylate copolymer), anionic polysaccharides, alone or as mixtures.

The composition of the invention has a pleasant, non-tacky and non-greasy texture, and is easy to apply to the entire head of hair to be styled. It also gives the hair flexibility and volume, while at the same time conserving a natural appearance and a soft, smooth feel.

In contrast with the compositions of the prior art, the head of hair thus styled is held without being made rigid, and the styling effects afforded by the composition of the invention are long-lasting throughout the day, or even for several days.

A subject of the present invention is also a cosmetic process for treating keratin fibres, in particular human keratin fibres such as the hair, comprising a step of applying a composition as defined previously to said keratin fibres.

The present invention also relates to the use of a composition as defined previously for styling, i.e. shaping and/or fixing, keratin fibres, in particular human keratin fibres such as the hair.

Other subjects, characteristics, aspects and advantages of the invention will emerge even more clearly on reading the description and the examples that follow.

In the text hereinbelow, unless otherwise indicated, the limits of a range of values are included in that range, notably in the expressions "between" and "ranging from ... to ...".

Moreover, the expression "at least one" used in the present description is equivalent to the expression "one or more".

### Non-aqueous dispersion of polymer particles

The composition according to the present invention comprises one or more dispersions of particles of at least one polymer, stabilized with one or more stabilizers in a non-aqueous medium.

The dispersions that may be used according to the invention consist of particles, which are generally spherical, of at least one polymer surface-stabilized with at least one stabilizer, in a non-aqueous medium.

These dispersions may notably be in the form of polymer nanoparticles in stable dispersion in a non-aqueous medium. The nanoparticles preferably have a number-mean size of between 5 and 600 nm, more preferentially between 10 and 500 nm and better still between 15 and 450 nm, given that beyond about 600 nm, the particle dispersions become much less stable.

The polymer particles of the dispersion preferably have a number-mean size ranging from 50 to 500 nm, more preferentially from 75 to 400 nm and better still from 100 to 250 nm.

Notably, these particles remain in the form of elementary particles, without forming agglomerates, when they are in dispersion in said non-aqueous media.

The polymers according to the invention may be linear, branched or even star homopolymers or copolymers. They may be random or alternating polymers. Preferably, they are linear random copolymers.

The polymer used in the particle dispersions according to the invention is an ethylenic polymer.

For the purposes of the present invention, the term "ethylenic polymer" means a polymer obtained by polymerization of at least two identical or different monomers, comprising an ethylenic unsaturation.

Said ethylenic polymer may be chosen by a person skilled in the art as a function of its properties. These polymers may in particular be crosslinked.

More particularly, said ethylenic polymer has at least one glass transition temperature (Tg) of less than or equal to -20°C, preferably between -150°C and -20°C, notably between -100°C and -25°C, preferentially between -95°C and -30°C, or even between -80°C and -35°C, and better still between -70°C and -40°C.

Preferably, the polymer has only one glass transition temperature.

The polymer may, however, have several glass transition temperatures, notably two Tg values; in this case, the lowest Tg value must be below -20°C.

In the present invention, the Tg values (or glass transition temperatures) indicated are theoretical Tg values determined from the theoretical Tg values of the constituent monomers of the polymer, which may be found in a reference manual such as the Polymer Handbook, 3rd Edition, 1989, John Wiley.

In the present description, the term "monomer with a Tg" means the monomer whose homopolymer has such a glass transition temperature.

In general, the monomers with a Tg of less than or equal to -20°C may represent from 50% to 100% by weight relative to the total weight of the initial monomers.

In a first embodiment of the invention, the polymers present in the dispersion may be derived from the polymerization of one or more monomers with a Tg of less than or equal to -20°C, preferably between -150°C and -20°C, notably between -100°C and -25°C, preferentially between -95°C and -30°C, or even between -80°C and -40°C, and better still between -70°C and -45°C.

In this embodiment, the monomer with a Tg of less than or equal to -20°C, or a mixture thereof, represents 100% by weight relative to the total weight of the initial monomers.

In a second embodiment of the invention, the polymers present in the dispersion may be derived from the polymerization of one or more monomers with a Tg of less than or equal to -20°C, and of one or more "additional" monomers with a Tg of greater than -20°C, but present in an amount such that the overall Tg of the polymer is less than or equal to -20°C.

For example, it is possible to combine in the final polymer a monomer with a Tg of the order of 100°C, which may be present in a proportion of 10-20% by weight relative to the total weight of monomers, and a monomer with a Tg of the order of -50°C, which may be present in a proportion of 80-90% by weight, so as to obtain a polymer with a Tg from about -40°C to -30°C.

In this embodiment, preferably, the additional monomer, or the mixture of such monomers, may be present in a proportion of from 0.01% to 50% by weight, relative to the total weight of the monomers, notably from 0.1% to 40% by weight, or even from 1% to 30% by weight, or from 5% to 15% by weight. The monomer with a Tg of less than or equal to -20°C, or the mixture of such monomers, may then be present in a proportion of from 50% to 99.99% by weight, notably from 60% to 99.9% by weight, or even from 70% to 99% by weight, or even from 85% to 95% by weight, relative to the total weight of monomers.

A person skilled in the art will know, on the basis of Fox's law and his general knowledge, how to determine the maximum amounts of additional monomer that may be present in the polymer of the dispersion, so as always to obtain at the end a polymer dispersion with a Tg of less than or equal to -20°C.

Moreover, the polymer present in the particle dispersion preferably comprises one or more "hydrophobic" monomers as defined below.

Thus, preferably, the ethylenic polymer according to the invention comprises 40% to 100% by weight, relative to the total weight of monomers, notably from 60% to 99% by weight, or even from 70% to 98% by weight, and better still from 60% to 95% by weight of hydrophobic monomer, alone or as a mixture.

This or these hydrophobic monomers may be chosen from monomers with a Tg of less than or equal to -20°C and/or from monomers with a Tg of greater than -20°C.

Preferably, the hydrophobic monomer(s) have a Tg of less than -20°C.

For the purposes of the present invention, the term "hydrophobic monomer" means a monomer having a value of the logarithm of the apparent 1-octanol/water partition coefficient, also known as log P, of greater than or equal to 2, for example between 2 and 11, preferably between 2.5 and 10, notably between 3 and 8, or even between 3.5 and 5.

The log P values are known and are determined according to a standard test which determines the concentration of the monomer in 1-octanol and water.

The values may also be calculated using the ACD (Advanced Chemistry Development) software Solaris V4.67; they may also be obtained from Exploring QSAR: hydrophobic, electronic and steric constants (ACS professional reference book, 1995). A website also exists which provides estimated values (address: http://esc.syrres.com/interkow/kowdemo.htm).

The log P value of certain common monomers are indicated below, determined using the ACD software:

**[Table 1]**

| | acrylate (* acrylamide) | methacrylate (* methacrylamide) |
|---|---|---|
| methyl (meth)acrylate | 0.793 ± 0.223 | 1.346 ± 0.250 |
| ethyl (meth)acrylate | 1.325 ± 0.223 | 1.877 ± 0.250 |
| propyl (meth)acrylate | 1.856 ± 0.223 | 2.408 ± 0.250 |
| isopropyl (meth)acrylate | 1.672 ± 0.228 | 2.224 ± 0.254 |
| n-butyl (meth)acrylate | 2.387 ± 0.223 | 2.940 ± 0.250 |
| isobutyl (meth)acrylate | 2.208 ± 0.228 | 2.756 ± 0.254 |
| tert-butyl(meth)acrylate | 2.022 ± 0.238 | 2.574 ± 0.261 |
| cyclohexyl (meth)acrylate | 2.853 ± 0.226 | 3.405 ± -0.252 |
| octyl (meth)acrylate | 4.513 ± 0.224 | 5.065 ± 0.521 |
| lauryl (meth)acrylate | 6.638 ± 0.224 | 7.190 ± 0.251 |
| tridecyl (meth)acrylate | 7.170 ± 0.224 | 7.712 ± 0.251 |
| cetyl (meth)acrylate | 8.764 ± 0.224 | 9.316 ± 0.251 |
| palmityl (meth)acrylate | >9 | >9 |
| stearyl (meth)acrylate | 9.826 ± 0.224 | 10.379 ± 0.251 |
| behenyl (meth)acrylate | 12.504 ± 0.251 | 11.952 ± 0.225 |
| oleyl (meth)acrylate | 9.308 ± 0.232 | >9 |
| tetrahydrofurfuryl (meth)acrylate | 0.800 ± 0.263 | 1.352 ± 0.283 |
| 2-ethylhexyl (meth)acrylate | 4.329 ± 0.229 | 4.881 ± 0.254 |
| 2-hydroxyethyl (meth)acrylate | 0.166 ± 0.258 | 0.718 ± 0.277 |
| ethoxyethyl (meth)acrylate | 1.335 ± 0.268 | 1.887 ± 0.293 |
| hydroxypropyl (meth)acrylate | 0.383 ± 0.241 | |
| dimethylaminoethyl (meth)acrylate (MADAME) | | 0.97 |
| N-isopropyl(meth)acrylamide * | 0.195 ± 0.256 | 0.748 ± 0.276 |
| N-octyl(meth)acrylamide * | 3.036 ± 0.253 | 3.558 ± 0.273 |
| N-tert-butyl(meth)acrylamide * | 1.02 | |
| N,N-dimethyl(meth)acrylamide* | -0.168 ± 0.556 | 0.906 ± 0.553 |
| N,N-dibutyl(meth)acrylamide* | 3.021 ± 0.557 | 3.573 ± 0.570 |
| (meth)acrylic acid | 0.35 | 0.83 |

Among the monomers with a Tg of less than or equal to -20°C that may be used to form the dispersion according to the invention, mention may be made of:
(i) the acrylic acid esters of formula CH₂=CHCOOR₁ with R₁ representing (a) a linear or branched, saturated or unsaturated carbon-based and notably hydrocarbon-based (alkyl) chain containing 2 to 12 carbon atoms, optionally comprising one or more intercalated heteroatoms chosen from O, N and S; and/or optionally substituted with one or more substituents chosen from -OH and halogen atoms (Cl, Br, I and F), with the exclusion of the tert-butyl chain; or alternatively R₁ representing (b) a polyoxyethylene group comprising from 5 to 30 ethylene oxide units; or else R₁ representing (c) a group -R-(OC₂H₄)n-H with R = C₁-C₁₂ alkyl and n is an integer between 5 and 30 inclusive.
   Mention may thus be made of ethyl, propyl, n-butyl, isobutyl, 2-ethylhexyl, octyl, isooctyl, isodecyl, decyl, lauryl, tridecyl, hydroxyethyl and hydroxypropyl acrylates.
(ii) the methacrylic acid esters of formula CH₂=C(CH₃)COOR₂ with R₂ representing (a) a linear or branched, saturated or unsaturated carbon-based and notably hydrocarbon-based (alkyl) chain containing 8 to 12 carbon atoms, optionally comprising one or more intercalated heteroatoms chosen from O, N and S; and/or optionally substituted with one or more substituents chosen from -OH and halogen atoms (Cl, Br, I and F); or alternatively R₂ representing (b) a polyoxyethylene group comprising from 5 to 30 ethylene oxide units; or else R₂ representing (c) a group -R-(OC₂H₄)n-H with R = C₁-C₃₀ alkyl and n is an integer between 5 and 30 inclusive.
   Mention may thus be made of octyl, isooctyl, decyl, isodecyl, dodecyl, lauryl, tridecyl, myristyl, cetyl, palmityl, stearyl, behenyl and oleyl methacrylates.
(iii) the vinyl esters of formula CH₂=CH-OCO-R₃ with R₃ representing a linear or branched, saturated or unsaturated carbon-based and notably hydrocarbon-based chain containing 2 to 12 carbon atoms, among which mention may be made of vinyl butyrate (or butanoate), vinyl ethylhexanoate, vinyl neononanoate and vinyl neododecanoate;
(iv) the vinyl esters of formula CH₂=CHOR₄ with R₄ representing a linear or branched, saturated or unsaturated carbon-based and notably hydrocarbon-based chain containing 1 to 12 carbon atoms, among which mention may be made of vinyl ether, methyl vinyl ether, ethyl vinyl ether, ethylhexyl vinyl ether and butyl vinyl ether;
(v) the N-alkyl(meth)acrylamides of formula CH₂=CHCONR₅R'₅ or CH₂=C(CH₃)CONR₅R'₅ with R₅ and R'₅ representing, independently of each other, a hydrogen atom or a linear, cyclic or branched, saturated or unsaturated, optionally aromatic (aryl, aralkyl or alkylaryl) carbon-based and notably hydrocarbon-based chain containing 6 to 28 carbon atoms, optionally comprising one or more intercalated heteroatoms chosen from O, N and S; and/or optionally substituted with one or more substituents chosen from -OH and halogen atoms (Cl, Br, I and F); given that at least one of the radicals R₅ and R'₅ is other than hydrogen; among which mention may be made of N-octylacrylamide and N-octadecylacrylamide.

The monomers with a Tg of less than or equal to -20°C that are more particularly preferred are ethyl acrylate, n-butyl acrylate, ethylhexyl acrylate, isobutyl acrylate, isooctyl acrylate, and a mixture thereof.

Among the monomers with a Tg of less than or equal to -20°C which are also hydrophobic, mention may be made of n-butyl, isobutyl, 2-ethylhexyl, octyl, isooctyl, isodecyl, decyl and lauryl acrylates; and also octyl, isooctyl, decyl, isodecyl, dodecyl, lauryl, tridecyl, cetyl, palmityl, stearyl and oleyl methacrylates.

Even more preferentially, the monomer with a Tg of less than or equal to -20°C is 2-ethylhexyl acrylate.

As mentioned above, the polymer in dispersion according to the invention may also comprise one or more additional monomers, with a Tg of greater than -20°C, preferably greater than 0°C, with the proviso that this or these additional monomers, and/or the amount thereof, are chosen such that the overall Tg value of the polymer is less than or equal to -20°C.

These additional monomers may be chosen from the following monomers:
(i) the vinyl compounds of formula CH₂=CHR₆ in which R₆ is:
   - a hydroxyl group;
   - a linear or branched alkyl group comprising 1 to 25 carbon atoms, in which one or more heteroatoms chosen from O, N, S and P are optionally intercalated, said alkyl group also possibly being optionally substituted with one or more substituents chosen from -OH and halogen atoms (Cl, Br, I and F);
   - a C₃ to C₈ cycloalkyl group such as cyclohexane,
   - a C₆ to C₂₀ aryl group such as phenyl,
   - a C₇ to C₃₀ aralkyl group (C₁-C₄ alkyl group) such as 2-phenylethyl or benzyl,
   - a 4- to 12-membered heterocyclic group containing one or more heteroatoms chosen from O, N and S,
   - a heterocycloalkyl group (alkyl of 1 to 4 carbons), such as furfuryl, furfurylmethyl or tetrahydrofurfurylmethyl,
said cycloalkyl, aryl, aralkyl, heterocyclic or heterocycloalkyl groups possibly being optionally substituted with one or more substituents chosen from a hydroxyl group, halogen atoms and linear or branched C₁-C₄ alkyl groups in which are optionally intercalated one or more heteroatoms chosen from O, N, S and P, said alkyl groups also possibly being optionally substituted with one or more substituents chosen from -OH and halogen atoms (Cl, Br, I and F).

Examples of vinyl monomers are vinylcyclohexane, styrene and vinyl acetate.

(ii) the acrylates of formula CH₂=CHCOOR₇ in which R₇ is a tert-butyl group, a C₃ to C₈ cycloalkyl group; a C₆ to C₂₀ aryl group; a C₇ to C₃₀ aralkyl group (C₁ to C₄ alkyl group); a 4- to 12-membered heterocyclic group containing one or more heteroatoms chosen from O, N and S; a heterocycloalkyl group (C₁ to C₄ alkyl) such as a furfuryl group; said cycloalkyl, aryl, aralkyl, heterocyclic or heterocycloalkyl groups possibly being optionally substituted with one or more substituents chosen from a hydroxyl group, halogen atoms and linear or branched C₁-C₄ alkyl groups in which are optionally intercalated one or more heteroatoms chosen from O, N, S and P, said alkyl groups also possibly being optionally substituted with one or more substituents chosen from a hydroxyl group and halogen atoms (Cl, Br, I and F).

Examples of such monomers are tert-butyl, t-butylcyclohexyl, t-butylbenzyl, furfuryl and isobornyl acrylates.
(iii) the methacrylates of formula CH₂=C(CH₃)COOR₃ in which R₈ is:
- a linear or branched carbon-based and notably hydrocarbon-based (alkyl) group containing 1 to 6 carbon atoms, such as a methyl, ethyl, propyl or isobutyl group, said alkyl group also possibly being optionally substituted with one or more substituents chosen from OH and halogen atoms (Cl, Br, I and F);
- a C₃ to C₈ cycloalkyl group;
- a C₆ to C₂₀ aryl group;
- a C₇ to C₃₀ aralkyl group (C₁-C₄ alkyl group);
- a 4- to 12-membered heterocyclic group containing one or more heteroatoms chosen from O, N and S;
- a heterocycloalkyl group (C₁-C₄ alkyl), such as a furfuryl group;
said cycloalkyl, aryl, aralkyl, heterocyclic or heterocycloalkyl groups possibly being optionally substituted with one or more substituents chosen from OH, halogen atoms and linear or branched C₁-C₄ alkyl groups in which are optionally intercalated one or more heteroatoms chosen from O, N, S and P, said alkyl groups also possibly being optionally substituted with one or more substituents chosen from hydroxyl groups and halogen atoms (Cl, Br, I and F).

Examples of such monomers are methyl, ethyl, propyl, n-butyl, isobutyl, t-butylcyclohexyl, t-butylbenzyl, methoxyethyl, methoxypropyl and isobornyl methacrylates.
(iv) the (meth)acrylamides of formula CH₂=CHCONR₉R'₉ or CH₂=C(CH₃)CONR₉R'₉ in which R₉ and R'₉, which may be identical or different, represent a hydrogen atom or a linear or branched C₁-C₅ alkyl group, such as an n-butyl, t-butyl or isopropyl group.

Examples of such monomers are N-butyl(meth)acrylamide, N-isopropyl(meth)acrylamide, N,N-dimethyl(meth)acrylamide and N,N-dibutyl(meth)acrylamide.

The monomers with a Tg of greater than -20°C that are more particularly preferred are furfuryl, isobornyl, tert-butyl, tert-butylcyclohexyl and tert-butylbenzyl acrylates; methyl, n-butyl, ethyl and isobutyl methacrylates, styrene, vinyl acetate and vinylcyclohexane, and mixtures thereof.

Among the monomers with a Tg of greater than -20°C, which are also hydrophobic, mention may be made of isobornyl, tert-butyl, tert-butylcyclohexyl and tert-butylbenzyl acrylates; n-butyl and isobutyl methacrylates, styrene.

The polymer present in the dispersion according to the invention may, obviously, comprise hydrophilic monomers (i.e. non-hydrophobic monomers or monomers with a log P of less than 2), which may have a Tg of less than or equal to -20°C or a Tg of greater than -20°C, or a mixture of such hydrophilic monomers.

Among the hydrophilic monomers that may be used, mention may be made of acrylic acid, methacrylic acid, dimethylaminoethyl methacrylate (DMAEMA), dimethylaminopropylmethacrylamide (DMAPMA), vinylpyridine and vinylimidazole, MADQUAT (or [2-(methacryloyloxy)ethyl]trimethylammonium chloride), MAPTAC (or methacrylamidopropyltrimethylammonium chloride), 2-hydroxyethyl methacrylate, 2-hydroxyethyl acrylate, N-tert-butylacrylamide, tetrahydrofurfuryl methacrylate, methyl methacrylate, ethyl methacrylate, methyl acrylate, ethyl acrylate, vinylpyrrolidone, vinyl acetate, the acrylates or methacrylates of formula CH₂=C(H,CH₃)COOR with R being an alkyl which may contain groups (OC₂H₄)ₘ-OR", with m = 5 to 150 and R" = H or C₁ to C₃₀ alkyl.

Preferably, the hydrophilic monomer is chosen from dimethylaminoethyl methacrylate.

When it includes acid functions, the polymer according to the invention may be neutralized with an organic base, for example a primary, secondary or tertiary amine, the amine possibly including (hydroxyl) substituents, such as amino-2-methyl-2-propanol, and the salified or quaternized forms thereof.

When it includes basic functions, the polymer according to the invention may be neutralized with organic acids which may include one or more carboxylic, sulfonic or phosphonic groups. They may be linear, branched or cyclic aliphatic acids, or alternatively aromatic acids. These acids may also include one or more heteroatoms chosen from O and N, for example in the form of hydroxyl groups. Mention may notably be made of acetic acid, α-hydroxyethanoic acid, α-hydroxyoctanoic acid, α-hydroxycaprylic acid, ascorbic acid, benzoic acid, behenic acid, capric acid, caproic acid, caprylic acid, citric acid, dodecylbenzenesulfonic acid, 2-ethylcaproic acid, folic acid, fumaric acid, galactaric acid, gluconic acid, glycolic acid, 2-hexadecyleicosanoic acid, hydroxycaproic acid, 12-hydroxystearic acid, isolauric (or 2-butyloctanoic) acid, isomyristic (or 2-hexyloctanoic) acid, isoarachidic (or 2-octyldodecanoic) acid, isolignoceric (or 2-decyltetradecanoic) acid, lactic acid, lauric acid, malic acid, myristic acid, oleic acid, palmitic acid, propionic acid, sebacic acid, stearic acid, tartaric acid, terephthalic acid, trimesic acid, undecylenic acid, propylbetaine, cocamidopropylbetaine, and mixtures thereof. Preferably, the polymer is neutralized with an organic carboxylic acid and notably 2-ethylcaproic, palmitic or decanoic acid.

The polymer of the particles used in the composition according to the invention is an ethylenic polymer and more preferentially a C₁-C₄ alkyl (meth)acrylate polymer.

The C₁-C₄ alkyl (meth)acrylate monomers may notably be chosen from methyl (meth)acrylate, ethyl (meth)acrylate, n-propyl (meth)acrylate, isopropyl (meth)acrylate, n-butyl (meth)acrylate and tert-butyl (meth)acrylate.

A C₁-C₄ alkyl acrylate monomer is advantageously used.

Preferentially, the polymer of the particles is a methyl acrylate and/or ethyl acrylate polymer.

The polymer of the particles may also comprise an ethylenically unsaturated acid monomer or the anhydride thereof, chosen notably from ethylenically unsaturated acid monomers comprising at least one carboxylic, phosphoric or sulfonic acid function, such as crotonic acid, itaconic acid, fumaric acid, maleic acid, maleic anhydride, styrenesulfonic acid, vinylbenzoic acid, vinylphosphoric acid, acrylic acid, methacrylic acid, acrylamidopropanesulfonic acid or acrylamidoglycolic acid, and salts thereof.

The ethylenically unsaturated acid monomer is preferably chosen from (meth)acrylic acid, maleic acid and maleic anhydride.

The salts may be chosen from salts of alkali metals, for example sodium or potassium; salts of alkaline-earth metals, for example calcium, magnesium or strontium; metal salts, for example zinc, aluminium, manganese or copper; ammonium salts of formula NH₄⁺; quaternary ammonium salts; salts of organic amines, for instance salts of methylamine, dimethylamine, trimethylamine, triethylamine, ethylamine, 2-hydroxyethylamine, bis(2-hydroxyethyl)amine or tris(2-hydroxyethyl)amine; lysine or arginine salts.

The polymer of the particles may thus comprise or consist essentially of from 80% to 100% by weight of C₁-C₄ alkyl (meth)acrylate and from 0 to 20% by weight of ethylenically unsaturated acid monomer other than the C₁-C₄ alkyl (meth)acrylates, relative to the total weight of the polymer.

According to a first embodiment of the invention, the polymer consists essentially of a polymer of one or more C₁-C₄ alkyl (meth)acrylate monomers.

According to a second embodiment of the invention, the polymer consists essentially of a copolymer of C₁-C₄ (meth)acrylate and of (meth)acrylic acid or maleic anhydride.

The polymer of the particles is advantageously chosen from:
- methyl acrylate homopolymers,
- ethyl acrylate homopolymers,
- methyl acrylate/ethyl acrylate copolymers,
- methyl acrylate/ethyl acrylate/acrylic acid copolymers,
- methyl acrylate/ethyl acrylate/maleic anhydride copolymers,
- methyl acrylate/acrylic acid copolymers,
- ethyl acrylate/acrylic acid copolymers,
- methyl acrylate/maleic anhydride copolymers,
- ethyl acrylate/maleic anhydride copolymers.

Advantageously, the polymer of the particles is a non-crosslinked polymer.

The polymers that may be used in the context of the present invention preferably have a number-average molecular weight (Mn) of between 2000 and 1 000 000, notably between 3000 and 800 000 and better still between 150 000 and 500 000.

The content of polymer in the form of particles in the dispersion is preferably between 5% and 80% by weight, and more preferentially between 20% and 60% by weight, stabilizer included, relative to the total weight of the dispersion.

The non-aqueous medium in which the polymer is dispersed may consist of any cosmetically or dermatologically acceptable oil, and in general any physiologically acceptable oil.

For the purposes of the present invention, the term "oil" means a fatty substance that is liquid at room temperature (25°C) and at atmospheric pressure (760 mmHg).

The term "fatty substance" refers to an organic compound that is insoluble in water at ordinary temperature (25°C) and at atmospheric pressure (760 mmHg or 1.013×10⁵ Pa) (solubility of less than 5%, preferably less than 1% and even more preferentially less than 0.1%). They bear in their structure at least one hydrocarbon-based chain including at least 6 carbon atoms or a sequence of at least two siloxane groups. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane.

Preferably, the non-aqueous medium comprises at least one oil chosen from carbon-based oils, hydrocarbon-based oils, fluoro oils and/or silicone oils of mineral, animal, plant or synthetic origin, and mixtures thereof.

Advantageously, use may be made of one or more oils that are volatile at room temperature and atmospheric pressure, for example having a non-zero vapour pressure at room temperature in particular ranging from 10⁻³ to 300 mmHg on condition that the boiling point is above 30°C. These volatile oils are favourable for producing a film with total "transfer-free" properties. After evaporation of these oils, a flexible, non-tacky film-forming deposit is obtained on the keratin fibres onto which the composition is applied. These volatile oils also facilitate the application of the composition to the keratin fibres.

These oils may be hydrocarbon-based oils or silicone oils optionally including alkyl or alkoxy groups at the end of the silicone chain, or as side groups.

As volatile silicone oils that can be used in the invention, mention may be made of linear or cyclic silicones containing from 2 to 7 silicon atoms, these silicones optionally including alkyl or alkoxy groups containing from 1 to 10 carbon atoms and also C₈-C₁₆ isoparaffins. These oils notably represent from 30% to 97.99% and better still from 30% to 75% of the total weight of the composition.

As volatile oil that may be used in the invention, mention may notably be made of octamethylcyclotetrasiloxane, decamethylcyclopentasiloxane, hexadecamethylcyclohexasiloxane, heptamethylhexyltrisiloxane, heptamethyloctyltrisiloxane or C₈-C₁₆ isoparaffins such as the Isopar and Permethyl products, and in particular isododecane.

In a particular embodiment of the invention, the non-aqueous medium is chosen from the group comprising:
- non-aqueous liquid compounds with a global solubility parameter according to the Hansen solubility space of less than 17 (MPa)^{1/2},

monoalcohols with a global solubility parameter according to the Hansen solubility space of less than or equal to 20 (MPa)^{1/2}, and
mixtures thereof.

The global solubility parameter δ according to the Hansen solubility space is defined in the article "Solubility parameter values" by Eric A. Grulke in the book "Polymer Handbook", 3rd Edition, Chapter VII, pages 519-559. The definition of solvents in the Hansen three-dimensional solubility space is described in the article by C.M. Hansen: "The three-dimensional solubility parameters", J. Paint Technol. 39, 105 (1967).

Among the non-aqueous media with a global solubility parameter according to the Hansen solubility space of less than or equal to 17 (MPa)^{1/2}, mention may be made of plant oils formed from fatty acid esters of polyols, in particular triglycerides, such as sunflower oil, sesame oil or rapeseed oil, or esters derived from acids or alcohols containing a long chain (i.e. a chain containing from 6 to 20 carbon atoms), notably the esters of formula RCOOR' in which R represents a higher fatty acid residue including from 7 to 19 carbon atoms and R' represents a hydrocarbon-based chain including from 3 to 20 carbon atoms, such as palmitates, adipates and benzoates, notably diisopropyl adipate.

Mention may also be made of hydrocarbons, and notably liquid paraffin, liquid petroleum jelly or hydrogenated polyisobutylene, isododecane or "Isopars", volatile isoparaffins.

Mention may also be made of silicone oils such as polydimethylsiloxanes and polymethylphenylsiloxanes, optionally substituted with aliphatic and/or aromatic groups, which are optionally fluorinated, or with functional groups such as hydroxyl, thiol and/or amine groups, and volatile silicone oils, which are notably cyclic.

Mention may also be made of the solvents, alone or as a mixture, chosen from (i) linear, branched or cyclic esters, containing more than 6 carbon atoms, (ii) ethers containing more than 6 carbon atoms, (iii) ketones containing more than 6 carbon atoms. The term "monoalcohols with a global solubility parameter according to the Hansen solubility space of less than or equal to 20 (MPa)^{1/2}" means aliphatic fatty alcohols containing at least 6 carbon atoms, the hydrocarbon-based chain not including any substitution groups.

Monoalcohols according to the invention that may be mentioned include oleyl alcohol, decanol, dodecanol, octadecanol and linoleyl alcohol.

Non-aqueous media that may also be used include those described in FR-A-2 710 646 from L.V.M.H.

Preferably, the aqueous medium in which the polymer is dispersed comprises at least one hydrocarbon-based oil.

For the purposes of the present invention, the term "hydrocarbon-based oil" means an oil formed essentially from, or even constituted of, carbon and hydrogen atoms, and optionally oxygen and nitrogen atoms, and not containing any silicon or fluorine atoms. It may contain alcohol, ester, ether, carboxylic acid, amine and/or amide groups.

The hydrocarbon-based oil is advantageously chosen from:
(i) hydrocarbon-based oils containing from 8 to 16 carbon atoms, and notably:
   - branched C₈-C₁₆ alkanes, for instance C₈-C₁₆ isoalkanes of petroleum origin (also known as isoparaffins), for instance isododecane (also known as 2,2,4,4,6-pentamethylheptane), isodecane, isohexadecane and, for example, the oils sold under the trade name Isopar or Permethyl,
   - linear alkanes, for instance n-dodecane (C12) and n-tetradecane (C14) sold by Sasol under the respective references Parafol 12-97 and Parafol 14-97, and also mixtures thereof, the undecane-tridecane mixture, the mixtures of n-undecane (C11) and of n-tridecane (C13) obtained in examples 1 and 2 of patent application WO 2008/155 059 from the company Cognis, and mixtures thereof,
(ii) short-chain esters (containing from 3 to 8 carbon atoms in total) such as ethyl acetate, methyl acetate, propyl acetate or n-butyl acetate;
(iii) hydrocarbon-based oils of plant origin such as triglycerides consisting of fatty acid esters of glycerol, the fatty acids of which may have chain lengths ranging from C₄ to C₂₄, these chains possibly being linear or branched, and saturated or unsaturated; these oils are notably heptanoic acid or octanoic acid triglycerides, or alternatively cereal germ oils such as wheatgerm oil, sunflower oil, grapeseed oil, sesame seed oil, corn oil, apricot oil, castor oil, shea oil, avocado oil, olive oil, soybean oil, sweet almond oil, palm oil, rapeseed oil, cotton oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion flower oil, beauty-leaf oil, arara oil or musk rose oil; shea butter; or else caprylic/capric acid triglycerides, for instance those sold by the company Stéarinerie Dubois or those sold under the names Miglyol 810^{®}, 812^{®} and 818^{®} by the company Dynamit Nobel;
(iv) synthetic ethers containing from 10 to 40 carbon atoms;
(v) linear or branched hydrocarbons of mineral or synthetic origin, such as petroleum jelly, polydecenes, hydrogenated polyisobutene such as Parleam^{®}, squalane, perhydrosqualene and liquid paraffins, and mixtures thereof;
(vi) synthetic esters such as oils of formula R₁COOR₂ in which R₁ represents a linear or branched fatty acid residue including from 1 to 40 carbon atoms and R₂ represents a, notably branched, hydrocarbon-based chain containing from 1 to 40 carbon atoms, on condition that R₁ + R₂ ≥ 10, for instance purcellin oil (cetostearyl octanoate), butyl stearate, 2-diethylhexyl succinate, isopropyl myristate, isopropyl palmitate, C₁₂ to C₁₅ alkyl benzoates, hexyl laurate, diisopropyl adipate, isononyl isononanoate, 2-ethylhexyl palmitate, isostearyl isostearate, 2-hexyldecyl laurate, 2-octyldecyl palmitate, 2-octyldodecyl myristate, alcohol or polyalcohol heptanoates, octanoates, decanoates or ricinoleates such as propylene glycol dioctanoate; hydroxylated esters such as isostearyl lactate, diisostearyl malate and 2-octyldodecyl lactate; polyol esters and pentaerythritol esters;
(vii) fatty alcohols that are liquid at room temperature, containing a branched and/or unsaturated carbon-based chain containing from 12 to 26 carbon atoms, for instance cetanol, stearyl alcohol, linoleyl alcohol, linolenyl alcohol, octyldodecanol, isostearyl alcohol, oleyl alcohol, 2-hexyldecanol, 2-butyloctanol or 2-undecylpentadecanol; and mixtures thereof.

Advantageously, the hydrocarbon-based oil is apolar (thus formed solely from carbon and hydrogen atoms).

The hydrocarbon-based oil is preferably chosen from hydrocarbon-based oils containing from 8 to 16 carbon atoms, in particular the apolar oils described previously.

Preferentially, the non-aqueous medium comprises a hydrocarbon-based oil, preferably chosen from hydrocarbon-based oils containing from 8 to 16 carbon atoms, more preferentially chosen from branched C₈-C₁₆ alkanes, and in particular isododecane.

The polymer dispersion may be manufactured as described in EP-A-749 747.

In general, a dispersion of polymer particles that is suitable for use in the invention may be prepared in the following manner, which is given as an example.

The polymerization may be performed in dispersion, i.e. by precipitation of the polymer during formation, with protection of the formed particles with a stabilizer.

In a first step, the stabilizer (or stabilizing polymer) is prepared by mixing the constituent monomer(s) of the stabilizing polymer with a free-radical initiator, in a solvent known as the synthesis solvent, and by polymerizing these monomers. In a second step, the constituent monomer(s) of the polymer of the particles are added to the stabilizing polymer formed and polymerization of these added monomers is performed in the presence of the free-radical initiator.

When the non-aqueous medium is a non-volatile hydrocarbon-based oil, the polymerization may be performed in an apolar organic solvent (synthesis solvent), followed by adding the non-volatile hydrocarbon-based oil (which should be miscible with said synthesis solvent) and selectively distilling off the synthesis solvent.

A synthesis solvent which is such that the monomers of the stabilizing polymer and the free-radical initiator are soluble therein, and the polymer particles obtained are insoluble therein, so that they precipitate therein during their formation, is thus chosen.

In particular, the synthesis solvent may be chosen from alkanes such as heptane or cyclohexane.

When the non-aqueous medium is a volatile hydrocarbon-based oil, the polymerization may be performed directly in said oil, which thus also acts as synthesis solvent. The monomers should also be soluble therein, as should the free-radical initiator, and the polymer of the particles which is obtained should be insoluble therein.

The monomers are preferably present in the synthesis solvent, before polymerization, in a proportion of 5-20% by weight. The total amount of the monomers may be present in the solvent before the start of the reaction, or a portion of the monomers may be added gradually as the polymerization reaction proceeds.

The free-radical initiator may notably be azobisisobutyronitrile or tert-butyl peroxy-2-ethylhexanoate.

The polymerization may be performed at a temperature ranging from 70 to 110°C.

The polymer particles are surface-stabilized, when they are formed during the polymerization, by means of the stabilizer. The stabilizer is a polymer other than the polymers a), b) and c).

The stabilization may be performed by any known means, and in particular by direct addition of the stabilizer, during the polymerization.

The stabilizer is preferably also present in the mixture before polymerization of the monomers of the polymer of the particles. However, it is also possible to add it continuously, notably when the monomers of the polymer of the particles are also added continuously.

In general, when a grafted polymer and/or a block polymer is used as stabilizer, the synthesis solvent is chosen such that at least some of the grafts or blocks of said polymer-stabilizer are soluble in said solvent, the rest of the grafts or blocks being insoluble therein. The polymer-stabilizer used during the polymerization should be soluble, or dispersible, in the synthesis solvent. Furthermore, a stabilizer including a portion (blocks, grafts or the like) which has a certain affinity for the polymer formed during the polymerization is preferably chosen.

When a random polymer is used as stabilizer, it is chosen such that it has a sufficient amount of groups making it soluble in the intended synthesis solvent.

Among the grafted polymers that may be mentioned are silicone polymers grafted with a hydrocarbon-based chain and hydrocarbon-based polymers grafted with a silicone chain.

Grafted copolymers having, for example, an insoluble backbone of polyacrylic type with soluble grafts of poly(12-hydroxystearic acid) type are also suitable for use.

Stabilizing polymers that may also be mentioned include:
(a) grafted or block copolymers comprising at least one block of polyorganosiloxane type and at least one block of a polymer (i) obtained from free-radical polymerization or (ii) obtained from a polycondensation, notably of polyether, polyester or polyamide type, or a mixture thereof, said copolymer possibly including fluoro species;

As grafted or block copolymers comprising at least one block of polyorganosiloxane type and at least one block of a free-radical polymer, mention may be made of grafted copolymers of acrylic/silicone type, which may notably be used when the non-aqueous medium is silicone-based.

When the grafted or block copolymers comprise at least one block of polyorganosiloxane type and at least one polyether block, the polyorganopolysiloxane block may notably be a polydimethylsiloxane or a poly(C₂-C₁₈)alkylmethylsiloxane; the polyether block may be a poly(C₂-C₁₈)-alkylene, in particular polyoxyethylene and/or polyoxypropylene. Use may thus be made of dimethicone copolyols or (C₂-C₁₈)alkyl methicone copolyols, which are optionally crosslinked. Use may be made, for example, of the dimethicone copolyol sold under the name Dow Corning 3225C by the company Dow Corning or the lauryl methicone copolyol sold under the name Dow Corning Q2-5200 by the company Dow Corning.

Mention may also be made of lauryl dimethicone copolyol crosspolymer, for example KSG31 or KSG32 from Shin-Etsu, cetyl dimethicone copolyol such as DMC 3071 from GE, and dimethicone copolyol PPG-3 oleyl ether such as KF-6026 from Shin-Etsu.

(b) block, grafted or sequenced copolymers of C₁-C₄ alkyl (meth)acrylates and of C₈-C₃₀ alkyl (meth)acrylates. Mention may be made of stearyl methacrylate/methyl methacrylate copolymer.

(c) grafted-block or block copolymers comprising at least one block resulting from the polymerization of ethylenic monomer, containing one or more optionally conjugated ethylenic bonds, and/or notably of dienes; and at least one polymer block derived from free-radical polymerization other than diene, notably derived from a vinyl, (meth)acrylic or (meth)acrylamide monomer, or from a polyether, a polyester or a polyamide, or mixtures thereof.

Use may notably be made of copolymers comprising at least one block resulting from the polymerization of at least one ethylenic monomer containing one or more optionally conjugated ethylenic bonds, such as ethylene, butadiene or isoprene, and of at least one block of a styrene polymer. When the ethylenic monomer includes several optionally conjugated ethylenic bonds, the residual ethylenic unsaturations after the polymerization are generally hydrogenated. Thus, in a known manner, the polymerization of isoprene leads, after hydrogenation, to the formation of an ethylene-propylene block, and the polymerization of butadiene leads, after hydrogenation, to the formation of an ethylene-butylene block. Among these block polymers that may be mentioned are copolymers of "diblock" or "triblock" type such as polystyrene/polyisoprene, polystyrene/polybutadiene such as those sold under the name "Luvitol HSB" by BASF, of the type such as polystyrene/copoly(ethylene-propylene) such as those sold under the name "Kraton" by Shell Chemical Co. or of the type such as polystyrene/copoly-(ethylene-butylene).

As grafted-block or block copolymers comprising at least one block resulting from the polymerization of at least one ethylenic monomer such as ethylene or isobutylene, and of at least one block of an acrylic polymer such as methyl methacrylate, mention may be made of poly(methyl methacrylate)/polyisobutylene diblock or triblock copolymers or grafted copolymers containing a poly(methyl methacrylate) backbone and polyisobutylene grafts.

As grafted-block or block copolymers comprising at least one block resulting from the polymerization of at least one ethylenic monomer and of at least one block of a polyether such as a C₂-C₁₈ polyalkylene, in particular polyoxyethylene and/or polyoxypropylene, mention may be made of polyoxyethylene/polybutadiene or polyoxyethylene/polyisobutylene diblock or triblock copolymers.

Stabilizers that may also be used include compounds such as:
(d) alkyl dimethicones in which the alkyl group comprises 6 to 32 carbon atoms, such as lauryl methicone and stearyl methicone, notably Si tec LDM 3107 from ISP, cetyl dimethicone such as Abil Wax 9801, behenoxy dimethicone such as Abil 5440 from Goldschmidt;
(e) the dimethiconol esters of formula: 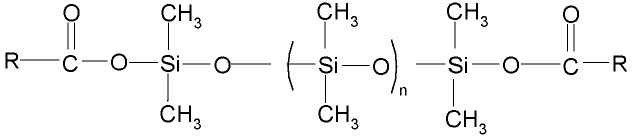 in which R is an alkyl radical containing 6 to 32 carbon atoms, such as dimethiconol behenate, and notably the products Ultrabee from Noveon and Pecosil DB from Phoenix Chemical;
(f) alkylamidoamines notably containing 6 to 60 carbon atoms, notably 12 to 50 carbon atoms, such as behenamidopropyldimethylamine and notably Catemol 220 from Phoenix Chemical, of formula: 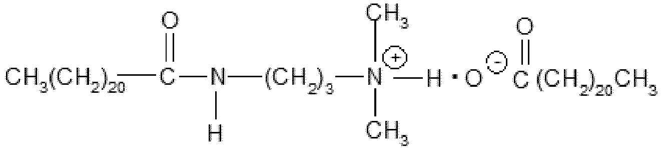
(g) copolymers comprising at least one polyorganosiloxane portion and fluoro groups, and notably fluoro silicones which may be represented by the formula: 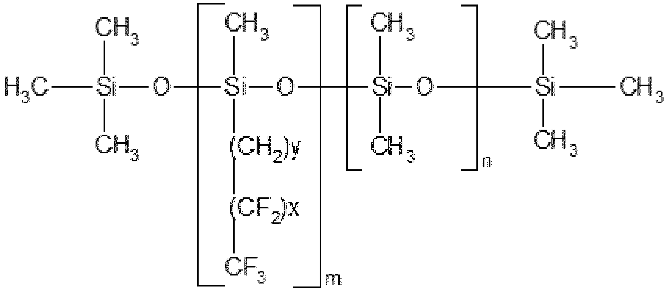 in which x is an integer between 3 and 12, preferably between 5 and 10, notably x = 8; y is an integer between 2 and 6, preferably 2 or 3; and m and n are such that the molecular weight of the compound is between 5000 and 15 000;
and more particularly perfluorononyl dimethicone, such as the products sold under the names Pecosil FSH-150 and 300 or Pecosil FSL-150 and 300 by Phoenix Chemical.

When the synthesis solvent is apolar, it is preferable to choose as stabilizer a polymer that provides the fullest possible coverage of the particles, several polymer-stabilizer chains then being adsorbed onto a particle of polymer obtained by polymerization.

In this case, it is preferred to use as stabilizer either a grafted polymer or a block polymer, so as to have better interfacial activity. Specifically, blocks or grafts that are insoluble in the synthesis solvent provide bulkier coverage at the surface of the particles.

When the liquid synthesis solvent comprises at least one silicone oil, the stabilizer is preferably chosen from the group consisting of grafted-block or block copolymers comprising at least one block of polyorganosiloxane type and at least one block of a free-radical polymer or of a polyether or of a polyester, for instance polyoxypropylene and/or polyoxyethylene blocks.

A plasticizer may be added to the polymer dispersion so as to lower the Tg of the polymers used. The plasticizer may be chosen from the plasticizers usually used in the field of application and notably from compounds liable to be solvents for the polymer. The plasticizer may be incorporated during the synthesis or added once the synthesis has been performed.

The stabilizer according to the invention is an isobornyl (meth)acrylate polymer chosen preferably from isobornyl (meth)acrylate homopolymer and statistical copolymers of isobornyl (meth)acrylate and of C₁-C₄ alkyl (meth)acrylate present in an isobornyl (meth)acrylate/C₁-C₄ alkyl (meth)acrylate weight ratio of greater than 4, preferably greater than 4.5 and even more advantageously greater than or equal to 5.

Advantageously, said weight ratio ranges from 4.5 to 19, preferably from 5 to 19 and more particularly from 5 to 12.

Thus, according to a particular embodiment, the composition according to the invention comprises one or more stabilizers, said stabilizer(s) being a statistical copolymer of isobornyl (meth)acrylate and of C₁-C₄ alkyl (meth)acrylate present in an isobornyl (meth)acrylate/C₁-C₄ alkyl (meth)acrylate weight ratio of greater than or equal to 5.

Advantageously, the stabilizer is chosen from:
- isobornyl acrylate homopolymers,
- statistical copolymers of isobornyl acrylate/methyl acrylate,
- statistical copolymers of isobornyl acrylate/methyl acrylate/ethyl acrylate, and
- statistical copolymers of isobornyl methacrylate/methyl acrylate,
in the weight ratio described previously.

The stabilizing polymer preferably has a number-average molecular weight ranging from 10 000 to 400 000 and preferably ranging from 20 000 to 200 000.

The polymer is in contact with the surface of the polymer particles and thus makes it possible to stabilize these particles at the surface, in particular in order to keep these particles in dispersion in the non-aqueous medium of the dispersion.

Advantageously, the combination of the stabilizer(s) + polymer(s) of the particles present in the dispersion comprises from 5% to 50% by weight of polymerized isobornyl (meth)acrylate and from 50% to 95% by weight of polymerized C₁-C₄ alkyl (meth)acrylate, relative to the total weight of the combination of the stabilizer(s) + polymer(s) of the particles.

Preferably, the stabilizer (or stabilizing polymer) is soluble in the non-aqueous medium, and is more preferentially soluble in the hydrocarbon-based oils, and better still soluble in isododecane.

From 10% to 30% by weight and preferably from 15% to 25% by weight of stabilizer may be used relative to the total weight of monomers used (stabilizer + polymer of the particles).

The polymer particle dispersion preferably comprises from 30% to 65% by weight and more preferentially from 40% to 60% by weight of solids, relative to the total weight of the dispersion.

Preferably, the total content of particle dispersion(s), present in the composition according to the invention, ranges from 0.1% to 20% by weight, more preferentially from 0.5% to 10% by weight and better still from 1% to 5% by weight relative to the total weight of the composition. For the purposes of the present invention, the term "content of particle dispersion(s)" means the sum of the contents of polymer(s), stabilizer(s) and non-aqueous medium.

### Cationic polymer

The composition according to the present invention also comprises one or more cationic polymers other than the polymers of the particle dispersion a).

For the purposes of the present invention, the term "cationic polymer" denotes any non-silicone polymer (not comprising any silicon atoms) containing cationic groups and/or groups that can be ionized into cationic groups.

The cationic polymers that may be used preferably have a weight-average molar mass (Mw) of between 500 and 5×10⁶ approximately and preferably between 10³ and 3×10⁶ approximately.

The cationic polymers according to the invention are chosen from homopolymers or copolymers including one or more units derived from vinylamine and optionally one or more units derived from vinylformamide, cationic polysaccharides, alkyldiallylamine or dialkyldiallylammonium cyclopolymers, and their mixtures

The present disclosure also refers to:
(1) homopolymers or copolymers derived from acrylic or methacrylic esters or amides and including at least one of the units having the following formulae: 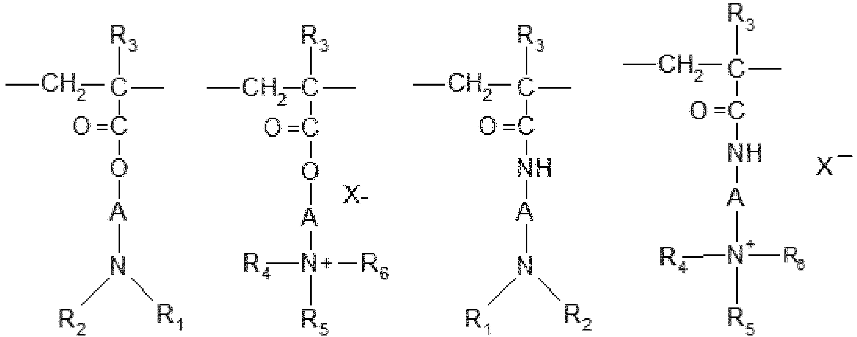 in which:
   - R₃, which may be identical or different, denote a hydrogen atom or a CH₃ radical;
   - A, which may be identical or different, represent a linear or branched divalent alkyl group of 1 to 6 carbon atoms, preferably 2 or 3 carbon atoms, or a hydroxyalkyl group of 1 to 4 carbon atoms;
   - R₄, R₅ and R₆, which may be identical or different, represent an alkyl group containing from 1 to 18 carbon atoms or a benzyl radical, and preferably an alkyl group containing from 1 to 6 carbon atoms;
   - R₁ and R₂, which may be identical or different, represent a hydrogen atom or an alkyl group containing from 1 to 6 carbon atoms, and preferably methyl or ethyl;
   - X denotes an anion derived from a mineral or organic acid, such as a methosulfate anion or a halide such as chloride or bromide.

The copolymers of family (1) may also contain one or more units derived from comonomers that may be chosen from the family of acrylamides, methacrylamides, diacetone acrylamides, acrylamides and methacrylamides substituted on the nitrogen with lower (C₁-C₄) alkyls, acrylic or methacrylic acid esters, vinyllactams such as vinylpyrrolidone or vinylcaprolactam, and vinyl esters.

Among these copolymers of family (1), mention may be made of:
- copolymers of acrylamide and of dimethylaminoethyl methacrylate quaternized with dimethyl sulfate or with a dimethyl halide, such as the product sold under the name Hercofloc by the company Hercules,
- copolymers of acrylamide and of methacryloyloxyethyltrimethylammonium chloride, such as the products sold under the name Bina Quat P 100 by the company Ciba Geigy,
- the copolymer of acrylamide and of methacryloyloxyethyltrimethylammonium methosulfate, such as the product sold under the name Reten by the company Hercules,
- quaternized or non-quaternized vinylpyrrolidone/dialkylaminoalkyl acrylate or methacrylate copolymers, such as the products sold under the name Gafquat by the company ISP, for instance Gafquat 734 or Gafquat 755, or alternatively the products known as Copolymer 845, 958 and 937. These polymers are described in detail in French patents 2 077 143 and 2 393 573;
- dimethylaminoethyl methacrylate/vinylcaprolactam/vinylpyrrolidone terpolymers, such as the product sold under the name Gaffix VC 713 by the company ISP;
- vinylpyrrolidone/methacrylamidopropyldimethylamine copolymers, such as the products sold under the name Styleze CC 10 by ISP;
- quaternized vinylpyrrolidone/dimethylaminopropylmethacrylamide copolymers such as the product sold under the name Gafquat HS 100 by the company ISP;
- polymers, preferably crosslinked polymers, of methacryloyloxy(C₁-C₄)alkyltri(C₁-C₄)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with an olefinically unsaturated compound, in particular methylenebisacrylamide. Use may be made more particularly of an acrylamide/methacryloyloxyethyltrimethylammonium chloride copolymer which is crosslinked or non-crosslinked (for example 20/80 by weight) notably in the form of a dispersion comprising 50% by weight of said copolymer in mineral oil. This dispersion is notably sold under the name Salcare^{®} SC 92 by the company Ciba. Use may also be made of a crosslinked or non-crosslinked methacryloyloxyethyltrimethylammonium chloride homopolymer, in particular dispersed at about 50% by weight in mineral oil or in a liquid ester. These dispersions are sold under the names Salcare^{®} SC 95 and Salcare^{®} SC 96 by the company Ciba.

(2) cationic polysaccharides, notably cationic celluloses and galactomannan gums. Among the cationic polysaccharides, mention may be made more particularly of cellulose ether derivatives including quaternary ammonium groups, cationic cellulose copolymers or cellulose derivatives grafted with a water-soluble quaternary ammonium monomer and cationic galactomannan gums.

The cellulose ether derivatives including quaternary ammonium groups are notably described in FR 1 492 597, and mention may be made of the polymers sold under the name Ucare Polymer JR (JR 400 LT, JR 125 and JR 30M) or LR (LR 400 and LR 30M) by the company Amerchol. These polymers are also defined in the CTFA dictionary as quaternary ammoniums of hydroxyethylcellulose that have reacted with an epoxide substituted with a trimethylammonium group.

Cationic cellulose copolymers or cellulose derivatives grafted with a water-soluble quaternary ammonium monomer are described notably in patent US 4 131 576, and mention may be made of hydroxyalkyl celluloses, for instance hydroxymethyl, hydroxyethyl or hydroxypropyl celluloses notably grafted with a methacryloylethyltrimethylammonium, methacrylamidopropyltrimethylammonium or dimethyldiallylammonium salt. The commercial products corresponding to this definition are more particularly the products sold under the names Celquat L 200 and Celquat H 100 by the company National Starch.

Mention may also be made of quaternized (poly)hydroxyethylcelluloses modified with groups including at least one fatty chain, such as alkyl, arylalkyl or alkylaryl groups including at least 8 carbon atoms, or mixtures thereof. The alkyl radicals borne by the above quaternized celluloses or hydroxyethylcelluloses preferably include from 8 to 30 carbon atoms. The aryl radicals preferably denote phenyl, benzyl, naphthyl or anthryl groups. Examples of quaternized alkylhydroxyethylcelluloses containing C₈-C₃₀ fatty chains that may be indicated include the products Quatrisoft LM 200^{®}, Quatrisoft LM-X 529-18-A^{®}, Quatrisoft LM-X 529-18-B^{®} (C₁₂ alkyl) and Quatrisoft LM-X 529-8^{®} (C₁₈ alkyl) sold by the company Amerchol, and the products Crodacel QM^{®}, Crodacel QL^{®} (C₁₂ alkyl) and Crodacel QS^{®} (C₁₈ alkyl) sold by the company Croda and the product Softcat SL 100^{®} sold by the company Amerchol.

The cationic galactomannan gums are described more particularly in patents US 3 589 578 and US 4 031 307, and mention may be made of guar gums comprising cationic trialkylammonium groups. Use is made, for example, of guar gums modified with a 2,3-epoxypropyltrimethylammonium salt (for example, a chloride). Such products are notably sold under the names Jaguar C13 S, Jaguar C 15, Jaguar C 17 and Jaguar C162 by the company Rhodia.

(3) polymers formed from piperazinyl units and divalent alkylene or hydroxyalkylene radicals containing linear or branched chains, optionally interrupted with oxygen, sulfur or nitrogen atoms or with aromatic or heterocyclic rings, and also the oxidation and/or quaternization products of these polymers.

(4) water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides can be alkylated or, if they include one or more tertiary amine functions, they can be quaternized;

(5) polyaminoamide derivatives resulting from the condensation of polyalkylene polyamines with polycarboxylic acids followed by alkylation with difunctional agents. Mention may be made, for example, of adipic acid/dialkylaminohydroxyalkyldialkylenetriamine polymers in which the alkyl radical includes from 1 to 4 carbon atoms and preferably denotes methyl, ethyl or propyl. Among these derivatives, mention may be made more particularly of the adipic acid/dimethylaminohydroxypropyl/diethylenetriamine polymers sold under the name Cartaretine F, F4 or F8 by the company Sandoz.

(6) polymers obtained by reacting a polyalkylene polyamine including two primary amine groups and at least one secondary amine group with a dicarboxylic acid chosen from diglycolic acid and saturated aliphatic dicarboxylic acids containing from 3 to 8 carbon atoms; the mole ratio between the polyalkylene polyamine and the dicarboxylic acid preferably being between 0.8:1 and 1.4:1; the resulting polyaminoamide being reacted with epichlorohydrin in a mole ratio of epichlorohydrin relative to the secondary amine group of the polyaminoamide preferably of between 0.5:1 and 1.8:1. Polymers of this type are sold in particular under the name Hercosett 57 by the company Hercules Inc. or else under the name PD 170 or Delsette 101 by the company Hercules in the case of the adipic acid/epoxypropyl/diethylenetriamine copolymer.

(7) cyclopolymers of alkyldiallylamine or of dialkyldiallylammonium, such as the homopolymers or copolymers including, as main constituent of the chain, units corresponding to the following formulae: 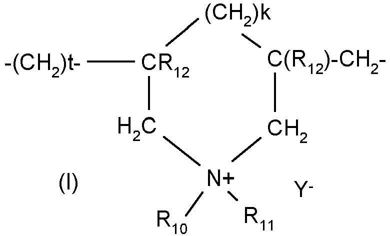 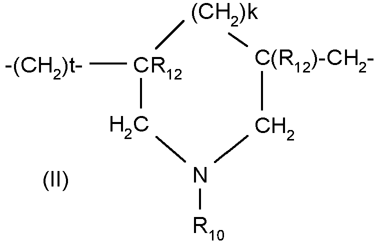 in which
- k and t are equal to 0 or 1, the sum k + t being equal to 1;
- R₁₂ denotes a hydrogen atom or a methyl radical;
- R₁₀ and R₁₁, independently of each other, denote a C₁-C₆ alkyl group, a C₁-C₅ hydroxyalkyl group, a C₁-C₄ amidoalkyl group; or alternatively R₁₀ and R₁₁ may denote, together with the nitrogen atom to which they are attached, a heterocyclic group such as piperidinyl or morpholinyl; R₁₀ and R₁₁, independently of each other, preferably denote a C₁-C₄ alkyl group;
- Y⁻ is an anion such as bromide, chloride, acetate, borate, citrate, tartrate, bisulfate, bisulfite, sulfate or phosphate.

Mention may be made more particularly of the homopolymer of dimethyldiallylammonium salts (for example chloride) for example sold under the name Merquat 100 by the company Nalco and the copolymers of diallyldimethylammonium salts (for example chloride) and of acrylamide, notably sold under the name Merquat 550 or Merquat 7SPR.

(8) quaternary diammonium polymers comprising repeating units of the following formula: 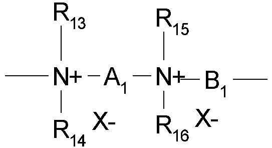 in which:
- R₁₃, R₁₄, R₁₅ and R₁₆, which may be identical or different, represent aliphatic, alicyclic or arylaliphatic radicals comprising from 1 to 20 carbon atoms or C₁-C₁₂ hydroxyalkyl aliphatic radicals;
   or else R₁₃, R₁₄, R₁₅ and R₁₆, together or separately, form, with the nitrogen atoms to which they are attached, heterocycles optionally comprising a second non-nitrogen heteroatom;
   or else R₁₃, R₁₄, R₁₅ and R₁₆ represent a linear or branched C₁-C₆ alkyl radical substituted with a nitrile, ester, acyl, amide or -CO-O-R₁₇-D or - CO-NH-R₁₇-D group, where R₁₇ is an alkylene and D is a quaternary ammonium group;
- A₁ and B₁ represent divalent polymethylene groups comprising from 2 to 20 carbon atoms which may be linear or branched, and saturated or unsaturated, and which may contain, linked to or inserted in the main chain, one or more aromatic rings, or one or more oxygen or sulfur atoms or sulfoxide, sulfone, disulfide, amino, alkylamino, hydroxyl, quaternary ammonium, ureido, amide or ester groups; and
- X⁻ denotes an anion derived from a mineral or organic acid;
   it being understood that A₁, R₁₃ and R₁₅ can form, with the two nitrogen atoms to which they are attached, a piperazine ring;
   in addition, if A₁ denotes a linear or branched, saturated or unsaturated alkylene or hydroxyalkylene radical, B₁ may also denote a group
   (CH₂)ₙ-CO-D-OC-(CH₂)ₚ-, with n and p, which may be identical or different, being integers ranging from 2 to 20, and D denoting:
      a) a glycol residue of formula -O-Z-O-, in which Z denotes a linear or branched hydrocarbon-based radical or a group corresponding to one of the following formulae: -(CH₂CH₂O)ₓ-CH₂CH₂- and -[CH₂CH(CH₃)O]_{y}-CH₂CH(CH₃)-, where x and y denote an integer from 1 to 4, representing a defined and unique degree of polymerization or any number from 1 to 4 representing an average degree of polymerization;
      b) a bis-secondary diamine residue, such as a piperazine derivative;
      c) a bis-primary diamine residue of formula -NH-Y-NH-, in which Y denotes a linear or branched hydrocarbon-based radical, or else the divalent radical -CH₂-CH₂-S-S-CH₂-CH₂-;
      d) a ureylene group of formula -NH-CO-NH-.

Preferably, X⁻ is an anion, such as chloride or bromide. These polymers have a number-average molar mass (Mn) generally of between 1000 and 100 000.

Mention may be made more particularly of polymers consisting of repeating units corresponding to the formula: 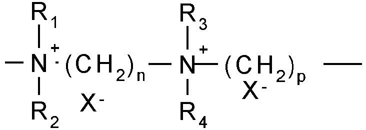 in which R₁, R₂, R₃ and R₄, which may be identical or different, denote an alkyl or hydroxyalkyl radical containing from 1 to 4 carbon atoms, n and p are integers ranging from 2 to 20, and X⁻ is an anion derived from a mineral or organic acid.

One compound that is particularly preferred is the one consisting of units for which R₁, R₂, R₃ and R₄ represent a methyl radical, n = 3, p = 6 and X = Cl, which is known as Hexadimethrine chloride according to the INCI (CTFA) nomenclature.
(9) polyquaternary ammonium polymers comprising units of the following formula: 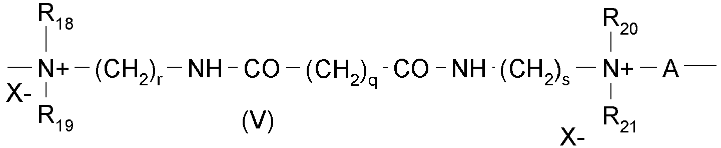 in which:
- R₁₈, R₁₉, R₂₀ and R₂₁, which may be identical or different, represent a hydrogen atom or a methyl, ethyl, propyl, β-hydroxyethyl, β-hydroxypropyl or
- CH₂CH₂(OCH₂CH₂)ₚOH radical, where p is equal to 0 or to an integer of between 1 and 6, with the proviso that R₁₈, R₁₉, R₂₀ and R₂₁ do not simultaneously represent a hydrogen atom,
- r and s, which may be identical or different, are integers between 1 and 6,
- q is equal to 0 or to an integer between 1 and 34,
- X⁻ denotes an anion, such as a halide,
- A denotes a divalent dihalide radical or preferably represents -CH₂-CH₂-O-CH₂-CH₂-.

Examples that may be mentioned include the products Mirapol^{®} A 15, Mirapol^{®} AD1, Mirapol^{®} AZ1 and Mirapol^{®} 175 sold by the company Miranol.

(10) quaternary polymers of vinylpyrrolidone and of vinylimidazole, for instance the products sold under the names Luviquat^{®} FC 905, FC 550 and FC 370 by the company BASF.

(11) polyamines such as Polyquart^{®} H sold by Cognis, which is referenced under the name Polyethylene Glycol (15) Tallow Polyamine in the CTFA dictionary.

(12) polymers including in their structure:
- one or more units corresponding to formula (A) below: 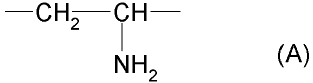
- and optionally one or more units corresponding to formula (B) below:

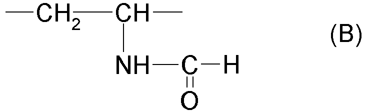

In other words, these polymers may be notably chosen from homopolymers or copolymers including one or more units derived from vinylamine and optionally one or more units derived from vinylformamide.

Preferably, these cationic polymers are chosen from polymers including, in their structure, from 5 mol% to 100 mol% of units corresponding to formula (A) and from 0 to 95 mol% of units corresponding to formula (B), preferentially from 10 mol% to 100 mol% of units corresponding to formula (A) and from 0 to 90 mol% of units corresponding to formula (B).

These polymers may be obtained, for example, by partial hydrolysis of polyvinylformamide. This hydrolysis may take place in acidic or basic medium.

The weight-average molecular mass of said polymer, measured by light scattering, may range from 1000 to 3 000 000 g/mol, preferably from 10 000 to 1 000 000 and more particularly from 100 000 to 500 000 g/mol.

The polymers including units of formula (A) and optionally units of formula (B) are notably sold under the name Lupamin by the company BASF, for instance, in a non-limiting manner, the products provided under the names Lupamin 9095, Lupamin 5095, Lupamin 1095, Lupamin 9030 (or Luviquat 9030) and Lupamin 9010.

(13) cationic polyvinyllactam polymers.

Such polymers are described, for example, in patent application WO-00/68282.

As cationic poly(vinyllactam) polymers according to the invention, vinylpyrrolidone/dimethylaminopropylmethacrylamide/dodecyldimethylmethac rylamidopropylammonium tosylate terpolymers, vinylpyrrolidone/dimethylaminopropylmethacrylamide/cocoyldimethylmethacr ylamidopropylammonium tosylate terpolymers, vinylpyrrolidone/dimethylaminopropylmethacrylamide/lauryldimethylmethacryl amidopropylammonium tosylate or chloride terpolymers are notably used.

(14) and mixtures of the above polymers.

Other cationic polymers that may be used in the context of the invention are cationic proteins or cationic protein hydrolysates, polyalkyleneimines, in particular polyethyleneimines, polymers comprising vinylpyridine or vinylpyridinium units, condensates of polyamines and of epichlorohydrin, quaternary polyureylenes and chitin derivatives such as chitosan.

Preferably, the cationic polymers according to the invention are chosen from those of families (1), (2), (7), (8) and (12), preferentially families (1), (2) and (7). Among the cationic polymers mentioned above, use may preferentially be made of cationic polysaccharides, notably quaternized (poly)hydroxyethylcelluloses modified with groups including at least one fatty chain, such as alkyl, arylalkyl or alkylaryl groups including at least 8 carbon atoms, or mixtures thereof. More particularly, the cationic polymer is chosen from hydroxyethylcelluloses which have reacted with a trimethylammonium epoxide and a lauryldimethylammonium epoxide (INCI name: Polyquaternium-67).

The content of the cationic polymer(s), present in the composition according to the invention, preferably ranges from 0.05% to 15% by weight, preferably from 0.5% to 10% by weight, and more preferentially from 1% to 5% by weight relative to the total weight of the composition.

The weight ratio between the total content of particle dispersion(s) and the total content of cationic polymer(s), present in the composition according to the invention, preferably ranges from 0.1 to 15, preferentially from 0.5 to 10 and better still from 1 to 5.

### Anionic polymer

The composition according to the present invention also comprises one or more anionic polymers other than the polymers of the particle dispersion a). The anionic polymers according to the invention are chosen from crosslinked or non-crosslinked acrylic or methacrylic acid homopolymers or copolymers and salts thereof, crosslinked or non-crosslinked 2-acrylamido-2-methylpropanesulfonic acid homopolymers and salts thereof and crosslinked or non-crosslinked acrylamide copolymers thereof and salts thereof, the branched block butyl acrylate/acrylic acid/ methacrylic acid anionic polymer (INCI name: AMP-acrylates/allyl methacrylate copolymer), anionic polysaccharides, alone or as mixtures.

For the purposes of the present invention, the term "anionic polymer" denotes any polymer containing anionic groups and/or groups that can be ionized into anionic groups and not containing any cationic groups and/or groups that can be ionized into cationic groups.

Anionic polymers that may be mentioned in the present disclosure as illustration include polymers including groups derived from carboxylic, sulfonic or phosphoric acids, and having a number-average molecular mass of between 500 and 5 000 000.

The carboxylic groups are provided by unsaturated monocarboxylic or dicarboxylic acid monomers, such as those corresponding to formula (I): 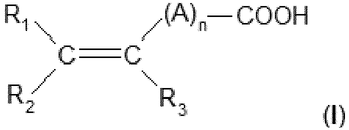 in which
n is an integer from 0 to 10,
A denotes a methylene group, optionally connected to the carbon atom of the unsaturated group or to the adjacent methylene group, when n is greater than 1, via a heteroatom, such as oxygen or sulfur,
R₁ denotes a hydrogen atom or a phenyl or benzyl group,
R₂ denotes a hydrogen atom, an alkyl group including from 1 to 4 carbon atoms or a carboxyl group,
R₃ denotes a hydrogen atom, an alkyl group including from 1 to 4 carbon atoms, a -CH₂-COOH group, a phenyl group or a benzyl group.

In formula (I) above, the alkyl group including from 1 to 4 carbon atoms may in particular denote methyl and ethyl groups.

The anionic polymers containing carboxylic or sulfonic groups that are preferred are:
A) copolymers of acrylic or methacrylic acid or salts thereof, including copolymers of acrylic acid and acrylamide, and methacrylic acid/acrylic acid/ethyl acrylate/methyl methacrylate copolymers, in particular Amerhold DR 25 sold by the company Amerchol, and sodium salts of polyhydroxycarboxylic acids. Mention may also be made of methacrylic acid/ethyl acrylate copolymers, notably in aqueous dispersion, such as Luviflex Soft and Luvimer MAE, which are sold by the company BASF.
B) copolymers of acrylic or methacrylic acids with a monoethylenic monomer such as ethylene, styrene, vinyl esters and acrylic or methacrylic acid esters, optionally grafted onto a polyalkylene glycol such as polyethylene glycol and optionally crosslinked. Such polymers are described in particular in French patent 1 222 944 and German patent application No. 2 330 956, the copolymers of this type including an optionally N-alkylated and/or hydroxyalkylated acrylamide unit in their chain as described notably in Luxembourg patent applications 75370 and 75371. Mention may also be made of copolymers of acrylic acid and of C₁-C₄ alkyl methacrylate.
   As another anionic fixing polymer from this class, mention may also be made of the butyl acrylate/acrylic acid/methacrylic acid branched block anionic polymer sold under the name Fixate G-100 L by the company Lubrizol (INCI name AMP-Acrylates/Allyl Methacrylate Copolymer).
C) crotonic acid-based copolymers, such as those including vinyl acetate or propionate units in their chain and optionally other monomers such as allylic esters or methallylic esters, vinyl ether or vinyl ester of a linear or branched saturated carboxylic acid with a long hydrocarbon-based chain, such as those including at least 5 carbon atoms, these polymers possibly being grafted and crosslinked, or alternatively a vinyl, allylic or methallylic ester of an α- or β-cyclic carboxylic acid. Such polymers are described, *inter alia,* in French patents 1 222 944, 1 580 545, 2 265 782, 2 265 781, 1 564 110 and 2 439 798. Commercial products that fall within this category are the resins 282930, 261314 and 281310 sold by the company National Starch.
   Mention may also be made, as copolymer derived from crotonic acid, of crotonic acid/vinyl acetate/vinyl tert-butylbenzoate terpolymers, and in particular Mexomer PW supplied by the company Chimex.
D) polymers derived from maleic, fumaric or itaconic acids or anhydrides with vinyl esters, vinyl ethers, vinyl halides, phenylvinyl derivatives, acrylic acid and its esters, these polymers may be esterified. Such polymers are described in particular in US patents 2 047 398, 2 723 248 and 2 102 113 and GB patent 839 805, and notably those sold under the names Gantrez^{®} AN or ES by the company ISP.
   Polymers also falling within this category are the copolymers of maleic, citraconic or itaconic anhydrides and of an allylic or methallylic ester optionally including an acrylamide or methacrylamide group, an α-olefin, acrylic or methacrylic esters, acrylic or methacrylic acids or vinylpyrrolidone in their chain, the anhydride functions being monoesterified or monoamidated. These polymers are described, for example, in French patents 2 350 384 and 2 357 241 by the Applicant.
E) polyacrylamides including carboxylate groups.
F) polymers comprising sulfonic groups. These polymers may be polymers including vinylsulfonic, styrenesulfonic, naphthalenesulfonic, acrylamidoalkylsulfonic or sulfoisophthalate units.

These polymers may notably be chosen from:
- polyvinylsulfonic acid salts with a molecular mass of between 1000 and 100 000 approximately, and also the copolymers with an unsaturated comonomer such as acrylic or methacrylic acids and esters thereof, and also acrylamide or derivatives thereof, vinyl ethers and vinylpyrrolidone;
- polystyrenesulfonic acid salts and sodium salts, with a molecular mass of about 500 000 and of about 100 000. These compounds are described in patent FR 2198719;
- polyacrylamidesulfonic acid salts such as those mentioned in patent US 4 128 631;

H) anionic polyurethanes, which may include silicone grafts and silicones bearing hydrocarbon-based grafts.

Examples of fixing polyurethanes that may notably be mentioned include the dimethylolpropionic acid/isophorone diisocyanate/neopentyl glycol/polyester diols copolymer (also known under the name polyurethane-1, INCI name) sold under the brand name Luviset^{®} Pur by the company BASF, and the dimethylolpropionic acid/isophorone diisocyanate/neopentyl glycol/polyester diols/silicone diamine copolymer (also known under the name polyurethane-6, INCI name) sold under the brand name Luviset^{®} Si PUR A by the company BASF.

Another anionic polyurethane that may also be used is Avalure UR 450. Polymers containing sulfoisophthalate groups, such as the polymers AQ55 and AQ48 sold by the company Eastman, may also be used.

Mention may also be made of crosslinked acrylic or methacrylic acid homopolymers or copolymers and salts thereof, crosslinked or non-crosslinked 2-acrylamido-2-methylpropanesulfonic acid homopolymers and salts thereof and crosslinked or non-crosslinked copolymers thereof, in particular acrylamide copolymers thereof and salts thereof, ammonium acrylate homopolymers or copolymers of ammonium acrylate and of acrylamide, alone or as mixtures.

Among the crosslinked acrylic or methacrylic acid homopolymers that may be mentioned are those crosslinked with an allyl alcohol ether of the sugar series, for instance the products sold under the names Carbopol 980, 981, 954, 2984 and 5984 by the company Noveon or the products sold under the names Synthalen M and Synthalen K by the company 3 VSA. These polymers have the INCI name Carbomer.

The crosslinked (meth)acrylic acid copolymers may be copolymers of (meth)acrylic acid and of C1-C6 alkyl (meth)acrylate, such as the polymer sold under the name Aqua SF1 by the company Noveon.

The crosslinked 2-acrylamido-2-methylpropanesulfonic acid homopolymers are notably sold under the name Hostacerin AMPS^{®} by the company Clariant.

Among the partially or totally neutralized crosslinked copolymers of 2-acrylamido-2-methylpropanesulfonic acid and of acrylamide, mention may be made in particular of the product described in Example 1 of EP 503 853, and reference may be made to said document as regards these polymers.

The composition may similarly comprise ammonium acrylate homopolymers or copolymers of ammonium acrylate and of acrylamide.

As examples of ammonium acrylate homopolymers, mention may be made of the product sold under the name Microsap PAS 5193 by the company Hoechst. Among the copolymers of ammonium acrylate and of acrylamide that may be mentioned is the product sold under the name Bozepol C Nouveau or the product PAS 5193 sold by the company Hoechst. Reference may be made notably to FR 2 416 723, US 2 798 053 and US 2 923 692 as regards the description and preparation of such compounds.

The anionic polymers may also be chosen from anionic polysaccharides notably such as acacia gums and carrageenans, in particular lambda-carrageenans.

According to the invention, the anionic polymers are preferably chosen from acrylic acid copolymers, such as the acrylic acid/ethyl acrylate/N-tert-butylacrylamide terpolymer sold under the name Ultrahold Strong^{®} by the company BASF, methacrylic acid/ethyl acrylate copolymers, notably in aqueous dispersion, such as Luviflex Soft and Luvimer MAE sold by the company BASF, crotonic acid-based copolymers, such as the vinyl acetate/vinyl tert-butylbenzoate/crotonic acid terpolymers and the crotonic acid/vinyl acetate/vinyl neododecanoate terpolymers sold under the name Resin 28-29-30 by the company National Starch, polymers derived from maleic, fumaric or itaconic acids or anhydrides with vinyl esters, vinyl ethers, vinyl halides, phenylvinyl derivatives, acrylic acid and its esters, such as the methyl vinyl ether/monoesterified maleic anhydride copolymer sold under the name Gantrez^{®} ES 425 by the company ISP, Luviset SI PUR, Mexomer PW, elastomeric or non-elastomeric anionic polyurethanes, polymers bearing sulfoisophthalate groups, and anionic polymers of the B) class, and even more particularly, use is preferably made of the butyl acrylate/acrylic acid/methacrylic acid branched block anionic polymer sold under the name Fixate G-100 L by the company Lubrizol (INCI name: AMP-Acrylates/Allyl Methacrylate Copolymer), crosslinked or non-crosslinked 2-acrylamido-2-methylpropanesulfonic acid homopolymers or copolymers, or salts thereof.

Use will preferably be made in the present invention of crosslinked or non-crosslinked 2-acrylamido-2-methylpropanesulfonic acid homopolymers or copolymers, or salts thereof, more preferentially crosslinked 2-acrylamido-2-methylpropanesulfonic acid homopolymers or salts thereof such as Hostacerin AMPS^{®} from Clariant (INCI name: Ammonium polyacryloyldimethyl taurate), anionic polysaccharides, in particular carrageenans and

The content of anionic polymer(s), present in the composition according to the invention, preferably ranges from 0.05% to 15% by weight, more preferentially from 0.1% to 10% by weight, and better still from 0.5% to 5% by weight, relative to the total weight of the composition.

The weight ratio between the total content of particle dispersion(s) and the total content of anionic polymer(s), present in the composition according to the invention, preferably ranges from 0.1 to 15, preferentially from 0.5 to 10 and better still from 1 to 5.

According to the invention, the cationic polymer(s)/anionic polymer(s) weight ratio preferably ranges from 10/90 to 90/10, better still from 20/80 to 80/20, in particular from 30/70 to 70/30 and more preferentially from 40/60 to 60/40.

Preferably, the composition according to the present invention is aqueous. The water is present in a content generally greater than or equal to 30% by weight relative to the total weight of the composition.

Preferably, the content of water present in the composition of the invention ranges from 30% to 98% by weight, preferably from 50% to 98% by weight and more preferentially from 65% to 95% by weight, relative to the total weight of the composition.

Besides water, predominantly contained in the aqueous phase, the composition according to the present invention may optionally comprise one or more organic solvents, or mixtures thereof.

Examples of organic solvents that may be mentioned include linear or branched C₂ to C₄ alkanols, such as ethanol and isopropanol; glycerol; polyols and polyol ethers, for instance 2-butoxyethanol, propylene glycol, hexylene glycol, dipropylene glycol, propylene glycol monomethyl ether, diethylene glycol monomethyl ether and monoethyl ether, and also aromatic alcohols or ethers, for instance benzyl alcohol or phenoxyethanol, and mixtures thereof.

The pH of the composition according to the invention generally ranges from 1.5 to 12, preferably from 2 to 7, preferentially from 2.5 to 7 and better still from 3 to 6.5.

The pH of the composition may be adjusted to the desired value by means of the basifying agents or acidifying agents that are customarily used. Among the basifying agents, examples that may be mentioned include aqueous ammonia, alkanolamines, and mineral or organic hydroxides. Among the acidifying agents, examples that may be mentioned include mineral or organic acids, for instance hydrochloric acid, orthophosphoric acid, sulfuric acid, carboxylic acids, for instance acetic acid, tartaric acid, citric acid or lactic acid, and sulfonic acids.

The composition according to the present invention may also optionally comprise one or more thickeners other than the above cationic and anionic polymers.

The composition according to the present invention may optionally also comprise one or more silicones other than the non-aqueous medium present in the particle dispersion(s) and the polymers described previously.

The silicones that may be used in the present invention may be solid or liquid, and volatile or non-volatile.

The silicones that may be used may be soluble or insoluble in the composition according to the invention; they may be in the form of oil, wax, resin or gum; silicone oils and gums are preferred.

Silicones are notably described in detail in Walter Noll's Chemistry and Technology of Silicones (1968), Academic Press.

The volatile silicones may be chosen from those with a boiling point of between 60°C and 260°C (at atmospheric pressure) and more particularly from:
i) cyclic polydialkylsiloxanes including from 3 to 7 and preferably 4 to 5 silicon atoms, such as
   - octamethylcyclotetrasiloxane and decamethylcyclopentasiloxane.
      Mention may be made of the products sold under the name Volatile Silicone 7207 by Union Carbide or Silbione 70045 V 2 by Rhodia, Volatile Silicone 7158 by Union Carbide or Silbione 70045 V 5 by Rhodia;
   - cyclocopolymers of the dimethylsiloxane/methylalkylsiloxane type having the chemical structure: with D : 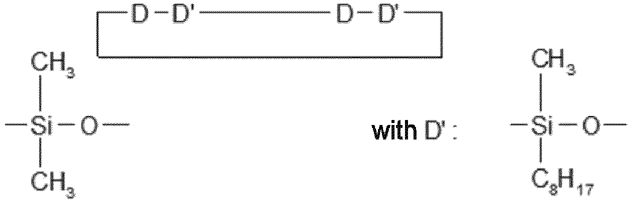 Mention may be made of Volatile Silicone FZ 3109 sold by the company Union Carbide;
   - mixtures of cyclic silicones with silicon-based organic compounds, such as the mixture of octamethylcyclotetrasiloxane and of tetratrimethylsilylpentaerythritol (50/50) and the mixture of octamethylcyclotetrasiloxane and of 1,1'-oxy(2,2,2',2',3,3'-hexatrimethylsilyloxy)bisneopentane;
ii) linear polydialkylsiloxanes containing 2 to 9 silicon atoms, which generally have a viscosity of less than or equal to 5 ×10⁻⁶ m²/s at 25°C, such as decamethyltetrasiloxane.

Other silicones belonging to this category are described in the article published in Cosmetics and Toiletries, Vol. 91, Jan. 76, pages 27-32, Todd & Byers Volatile Silicone Fluids for Cosmetics; mention may be made of the product sold under the name SH 200 by the company Toray Silicone.

Among the non-volatile silicones, mention may be made, alone or as a mixture, of polydialkylsiloxanes and notably polydimethylsiloxanes (PDMS), polydiarylsiloxanes, polyalkylarylsiloxanes, silicone gums and resins, and also organopolysiloxanes (or organomodified polysiloxanes, or alternatively organomodified silicones) which are polysiloxanes including in their structure one or more organofunctional groups, generally attached via a hydrocarbon-based group, and preferably chosen from aryl groups, amine groups, alkoxy groups and polyoxyethylene or polyoxypropylene groups.

The organomodified silicones may be polydiarylsiloxanes, notably polydiphenylsiloxanes, and polyalkylarylsiloxanes functionalized with the organofunctional groups mentioned previously. The polyalkylarylsiloxanes are particularly chosen from linear and/or branched polydimethyl/methylphenylsiloxanes and polydimethyl/diphenylsiloxanes.

Among the organomodified silicones, mention may be made of organopolysiloxanes including:
- polyoxyethylene and/or polyoxypropylene groups optionally including C₆-C₂₄ alkyl groups, such as dimethicone copolyols, and notably those sold by the company Dow Corning under the name DC 1248 or the oils Silwet^{®} L 722, L 7500, L 77 and L 711 from the company Union Carbide; or alternatively (C₁₂)alkylmethicone copolyols, and notably those sold by the company Dow Corning under the name Q2-5200;
- substituted or unsubstituted amine groups, in particular C₁-C₄ aminoalkyl groups; mention may be made of the products sold under the names GP4 Silicone Fluid and GP7100 by the company Genesee, or under the names Q2-8220 and DC929 or DC939 by the company Dow Corning;
- thiol groups, such as the products sold under the names GP 72 A and GP 71 from Genesee;
- alkoxylated groups, such as the product sold under the name Silicone Copolymer F-755 by SWS Silicones and Abil Wax^{®} 2428, 2434 and 2440 by the company Goldschmidt;
- hydroxylated groups, for instance polyorganosiloxanes bearing a hydroxyalkyl function;
- acyloxyalkyl groups, such as the polyorganosiloxanes described in patent US-A-4 957 732;
- anionic groups of the carboxylic acid type, as described, for example, in EP 186 507, or of the alkylcarboxylic type, such as the product X-22-3701 E from the company Shin-Etsu; or else of the 2-hydroxyalkylsulfonate or 2-hydroxyalkylthiosulfate type, such as the products sold by the company Goldschmidt under the names Abil^{®} S201 and Abil^{®} S255;
- hydroxyacylamino groups, such as the polyorganosiloxanes described in patent application EP 342 834; mention may be made, for example, of the product Q2-8413 from the company Dow Corning.

The silicones may also be chosen from polydialkylsiloxanes, among which mention may be made mainly of polydimethylsiloxanes bearing trimethylsilyl end groups. Among these polydialkylsiloxanes, mention may be made of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Corning, such as DC200 with a viscosity of 60 000 mm²/s;
- the Viscasil^{®} oils from General Electric and certain oils of the SF series (SF 96, SF 18) from General Electric.

Mention may also be made of polydimethylsiloxanes bearing dimethylsilanol end groups, known under the name dimethiconol (CTFA), such as the oils of the 48 series from the company Rhodia.

In this category of polydialkylsiloxanes, mention may also be made of the products sold under the names Abil Wax^{®} 9800 and 9801 by the company Goldschmidt, which are polydi(C1-C20)alkylsiloxanes.

Products that may be used more particularly in accordance with the invention are mixtures such as:
- mixtures formed from a polydimethylsiloxane with a hydroxy-terminated chain, or dimethiconol (CTFA), and from a cyclic polydimethylsiloxane, also known as cyclomethicone (CTFA), such as the product Q2-1401 sold by the company Dow Corning.

The polyalkylarylsiloxanes are particularly chosen from linear and/or branched polydimethyl/methylphenylsiloxanes and polydimethyl/diphenylsiloxanes with a viscosity ranging from 1×10⁻⁵ to 5×10⁻² m²/s at 25°C.

Among these polyalkylarylsiloxanes, mention may be made of the products sold under the following names:
- Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

The composition according to the present invention may also optionally comprise one or more additives, other than the compounds of the invention and among which mention may be made of nonionic, cationic, anionic, amphoteric or zwitterionic surfactants, and mixtures thereof, fatty substances other than the non-aqueous medium of the particle dispersion(s) and other than the silicones described previously, nonionic polymers, or mixtures thereof, antidandruff agents, anti-seborrhoea agents, vitamins and provitamins including panthenol, sunscreens, mineral or organic pigments, sequestrants, plasticizers, solubilizers, acidifying agents, opacifiers or nacreous agents, antioxidants, hydroxy acids, fragrances, preserving agents, pigments and ceramides.

According to the invention, the composition also comprises one or more additional salts, other than the compounds of the invention.

For the purposes of the present invention, the term "additional salts" means alkali metal salts such as sodium chloride; alkaline-earth metal salts such as CaCl₂; organic acid salts such as sodium citrate, sodium lactate, sodium acetate, and mixtures thereof; more preferentially alkali metal salts and in particular sodium chloride.

Needless to say, a person skilled in the art will take care to select this or these optional additional compounds such that the advantageous properties intrinsically associated with the composition according to the invention are not, or are not substantially, adversely affected by the envisaged addition(s).

The above additives may generally be present in an amount, for each of them, of between 0 and 20% by weight relative to the total weight of the composition.

The present invention also relates to a cosmetic treatment process, preferably a process for treating human keratin fibres such as the hair, notably a styling process, i.e. a process for fixing and/or shaping keratin fibres, in particular human keratin fibres such as the hair, comprising the application to said keratin fibres of a composition as defined previously.

The shaping process according to the invention may be performed on wet or dry, preferably wet, keratin fibres.

On conclusion of the process, the keratin fibres are advantageously rinsed with water before being dried or left to dry.

In a preferred embodiment of the invention, the process comprises:
(i) a step of applying to the keratin fibres a composition as defined in the appended claims comprising:
   (a) one or more dispersions of particles of at least one polymer, stabilized with one or more stabilizers in a non-aqueous medium,
   (b) one or more cationic polymers, and
   (c) one or more anionic polymers, followed by:
(ii) a step of rinsing the keratin fibres, preferably with water.

The present invention finally relates to the use of a composition as described above for the cosmetic treatment of, notably for styling, keratin fibres, in particular human keratin fibres such as the hair.

More particularly, the present invention relates to the use of a composition as described above for styling the hair, i.e. for shaping and/or fixing (holding) the hairstyle.

The examples that follow serve to illustrate the invention without, however, being limiting in nature.

### EXAMPLES

### Preparation example

In a first step, 1300 g of isododecane, 315.2 g of isobornyl acrylate, 12.5 g of methyl acrylate, 12.5 g of ethyl acrylate and 3.4 g of tert-butyl peroxy-2-ethylhexanoate (Trigonox 21S from Akzo) were placed in a reactor.

The mixture was heated to 90°C under argon with stirring and, after reaction, 540 g of isododecane and 360 g of ethyl acetate were added.

After 2 hours of reaction, 1430 g of isododecane were added to the reactor feedstock and the mixture was heated to 90°C.

In a second step, a mixture of 145 g of methyl acrylate, 934 g of ethyl acrylate, 157 g of acrylic acid, 12.36 g of Trigonox 21S, 741.6 g of isododecane and 494.4 g of ethyl acetate were added over 2 hours 30 minutes by pouring. After reaction, 3 liters of an isododecane/ethyl acetate mixture (60/40 weight/weight) were added and total evaporation of the ethyl acetate and partial evaporation of the isododecane was performed to obtain a solids content of 50% by weight.

A dispersion in isododecane of methyl acrylate/ethyl acrylate/acrylic acid copolymer particles stabilized with an isobornyl acrylate/methyl acrylate/ethyl acrylate statistical copolymer stabilizer was obtained.

The oily dispersion contains in total (stabilizer + particles) 10% acrylic acid, 10% methyl acrylate, 60% ethyl acrylate and 20% isobornyl acrylate.

The polymer particles of the dispersion have a number-mean size of about 160 nm.

### Composition examples

### Example 1

Compositions (A1), (A2) (A3), (A4), (A5), (A6) and (A7) were prepared using the ingredients of which the contents, expressed as mass percentages of active material, relative to the total weight of each composition, are mentioned in the table below.

**[Table 2]**

| | A1 (inv) | A2 | A3 | A4 | A5 | A6 | A7 |
|---|---|---|---|---|---|---|---|
| Ammonium polyacryloyldimethyl taurate (Hostacerin AMPS^{®}) | 1.615 | 1.615 | | 1.615 | | 1.615 | |
| Polyquaternium-67 (Softcat Polymer SL-100) | 1.29 | | 1.29 | 1.29 | | | 1.29 |
| Sodium chloride | 2 | 2 | 2 | 2 | 2 | 2 | 2 |
| Dispersion according to Preparation Example 1 at 50% in isododecane | 1.75 AM | | | | 1.75 AM | 1.75 AM | 1.75 AM |
| Water qs | 100 | 100 | 100 | 100 | 100 | 100 | 100 |

Each of the compositions (A1) to (A7) thus obtained was applied to 2.7 g locks of natural hair washed beforehand with a DOP shampoo, at a rate of 1 g of composition per lock.

The locks of hair were then rinsed with water for 15 seconds. The locks were then towel-dried and then laid flat and left to dry naturally.

After this treatment, the fixing and the shape hold were evaluated on the dry hair, by six trained experts:
- The fixing was evaluated by applying pressure to the lock between two fingers of the hand: the more difficult the lock of hair is to crack, the stronger the fixing
- The hold was evaluated by passing the fingers through the lock three times from the root to the end: the more the lock conserves its initial structure after passing the fingers through, the greater the hold.

These two parameters are graded from 0 to 5. The minimum limit of these two parameters is acquired after wetting an untreated lock with water: the fixing and hold are thus zero. The maximum limit is obtained after applying to the lock Vivelle Dop brand "béton" (ultra-strong hold) fixing styling gel: its fixing and hold parameters are each 5 out of 5.

### Results

**[Table 3]**

| | A1 (invention) | A2 | A3 | A4 | A5 | A6 | A7 |
|---|---|---|---|---|---|---|---|
| Fixing | 3 | 0 | 0 | 1.5 | 1.5 | 1.5 | 0 |
| Fixing persistence | 3 | 0 | 0 | 1.5 | 0.5 | 0.5 | 0 |

The locks treated with composition (A1) have good fixing and good fixing persistence.

Furthermore, the locks have a feel that is neither tacky nor greasy.

### Examples 2 to 5

Compositions 2 to 5 were prepared from the following ingredients.

**[Table 4]**

| | 2 | 3 | 4 | 5 |
|---|---|---|---|---|
| Ammonium polyacryloyldimethyl taurate | | 0.91 | 1.61 | |
| (Hostacerin AMPS^{®}) | | | | |
| AMP-acrylates/allyl methacrylate copolymer | 1.06 | | 1.06 | |
| (Fixate G100 L from Lubrizol) | | | | |
| Carrageenan (Satiagum VPC 430 from Cargill) | | | | 1.26 |
| Vinylamine/vinylformamide copolymer (Luviquat 9030 from BASF) | 0.94 | | | |
| Hydroxypropyl guar hydroxypropyltrimonium chloride (Jaguar C162 from Solvay) | | 1.09 | | |
| Polyquaternium-67(Softcat Polymer SL-100) | | | 1.29 | |
| Chitosan (Kionutrime CSG from Kitozyme) | | | | 0.74 |
| Sodium chloride | 10 | 2 | 10 | 5 |
| Dispersion according to Preparation Example 1 at 50% in isododecane | 2.5 AM | 2.5 AM | 2.5 AM | 2.5 AM |
| Water qs | 100 g | 100 g | 100 g | 100 g |

The locks treated with compositions 2 to 5 have good fixing and good fixing persistence.

## Claims

1. Composition comprising:
(a) one or more dispersions of particles of at least one polymer, stabilized with one or more polymer stabilizers in a non-aqueous medium,
(b) one or more cationic polymers, and
(c) one or more anionic polymers, wherein:
- the stabilizer is an isobornyl (meth)acrylate polymer,
- the polymer (a) in the form of particles is an ethylenic polymer,
- the cationic polymers are chosen from homopolymers or copolymers including one or more units derived from vinylamine and optionally one or more units derived from vinylformamide, cationic polysaccharides, alkyldiallylamine or dialkyldiallylammonium cyclopolymers, and their mixtures,
- the anionic polymers are chosen from crosslinked or non-crosslinked acrylic or methacrylic acid homopolymers or copolymers and salts thereof, crosslinked or non-crosslinked 2-acrylamido-2-methylpropanesulfonic acid homopolymers and salts thereof and crosslinked or non-crosslinked acrylamide copolymers thereof and salts thereof, the branched block butyl acrylate/acrylic acid/methacrylic acid anionic polymer (INCI name: AMP-acrylates/allyl methacrylate copolymer), anionic polysaccharides, alone or as mixtures.

2. Composition according to either of the preceding claims, **characterized in that** the polymer (a) is a C₁-C₄ alkyl (meth)acrylate polymer.

3. Composition according to any one of the preceding claims, **characterized in that** the polymer (a) comprises an ethylenically unsaturated acid monomer or the anhydride thereof, preferably chosen from (meth)acrylic acid, maleic acid and maleic anhydride.

4. Composition according to any one of the preceding claims, **characterized in that** the polymer (a) is chosen from:
- methyl acrylate homopolymers,
- ethyl acrylate homopolymers,
- methyl acrylate/ethyl acrylate copolymers,
- methyl acrylate/ethyl acrylate/acrylic acid copolymers,
- methyl acrylate/ethyl acrylate/maleic anhydride copolymers,
- methyl acrylate/acrylic acid copolymers,
- ethyl acrylate/acrylic acid copolymers,
- methyl acrylate/maleic anhydride copolymers,
- ethyl acrylate/maleic anhydride copolymers.

5. Composition according to any one of the preceding claims, **characterized in that** the non-aqueous medium comprises at least one oil chosen from carbon-based oils, hydrocarbon-based oils, fluoro oils and/or silicone oils of mineral, animal, plant or synthetic origin, and mixtures thereof.

6. Composition according to any one of the preceding claims, **characterized in that** the non-aqueous medium comprises at least one hydrocarbon-based oil, preferably chosen from:
(i) hydrocarbon-based oils containing from 8 to 16 carbon atoms;
(ii) short-chain esters containing from 3 to 8 carbon atoms in total;
(iii) hydrocarbon-based oils of plant origin;
(iv) synthetic ethers containing from 10 to 40 carbon atoms;
(v) linear or branched hydrocarbons, of mineral or synthetic origin;
(vi) synthetic esters;
(vii) fatty alcohols that are liquid at room temperature, with a branched and/or unsaturated carbon-based chain containing from 12 to 26 carbon atoms; and
(viii) mixtures thereof.

7. Composition according to any one of the preceding claims, **characterized in that** the stabilizer is an isobornyl (meth)acrylate polymer chosen from isobornyl (meth)acrylate homopolymer and statistical copolymers of isobornyl (meth)acrylate and of C₁-C₄ alkyl (meth)acrylate preferably present in an isobornyl (meth)acrylate/C₁-C₄ alkyl (meth)acrylate weight ratio of greater than 4, more preferentially greater than 4.5 and better still greater than or equal to 5.

8. Composition according to any one of the preceding claims, **characterized in that** the total content of particle dispersion(s) ranges from 0.1% to 20% by weight, more preferentially from 0.5% to 10% by weight and better still from 1% to 8% by weight relative to the weight of the composition.

9. Composition according to any one of the preceding claims, **characterized in that** the cationic polymers are chosen from cationic polysaccharides.

10. Composition according to any one of the preceding claims, **characterized in that** the content of the cationic polymer(s) ranges from 0.05% to 15% by weight, preferably from 0.5% to 10% by weight and more preferentially from 1% to 5% by weight, relative to the total weight of the composition.

11. Composition according to any one of the preceding claims, **characterized in that** the content of the anionic polymer(s) ranges from 0.05% to 15% by weight, more preferentially from 0.1% to 10% by weight and better still from 0.5% to 5% by weight, relative to the total weight of the composition.

12. Composition according to any one of the preceding claims, **characterized in that** the cationic polymer(s)/anionic polymer(s) weight ratio ranges from 10/90 to 90/10, better still from 20/80 to 80/20; preferably, this weight ratio ranges from 30/70 to 70/30 and more preferentially from 40/60 to 60/40.

13. Composition according to any one of the preceding claims, **characterized in that** the composition comprises water in a content ranging from 30% to 98% by weight, preferably from 50% to 98% by weight and more preferentially from 65% to 95% by weight, relative to the total weight of the composition.

14. Cosmetic process for treating keratin fibres, in particular human keratin fibres such as the hair, comprising the application to said keratin fibres of a composition as defined in any one of the preceding claims.

15. Use of a composition as defined in any one of Claims 1 to 13, for styling keratin fibres, in particular human keratin fibres such as the hair.

## Patentansprüche

1. Zusammensetzung, umfassend:
(a) eine oder mehrere Dispersionen von Partikeln mindestens eines Polymers, stabilisiert mit einem oder mehreren Polymerstabilisatoren in einem nichtwässrigen Medium,
(b) ein oder mehrere kationische Polymere, und
(c) ein oder mehrere anionische Polymere, wobei:
- der Stabilisator ein Isobornyl(meth)acrylat-Polymer ist,
- das Polymer (a) in Form von Partikeln ein ethylenisches Polymer ist,
- die kationischen Polymere ausgewählt sind aus Homopolymeren oder Copolymeren, einschließlich einer oder mehrerer Einheiten, abgeleitet von Vinylamin, und gegebenenfalls einer oder mehrerer Einheiten, abgeleitet von Vinylformamid, kationischen Polysacchariden, Alkyldiallylamin oder Dialkyldiallylammoniumcyclopolymeren, und deren Mischungen,
- die anionischen Polymere ausgewählt sind aus vernetzten oder unvernetzten Acryl- oder Methacrylsäure-Homopolymeren oder -Copolymeren und Salzen davon, vernetzten oder unvernetzten 2-Acrylamido-2-methylpropansulfonsäure-Homopolymeren und Salzen davon und vernetzten oder unvernetzten Acrylamid-Copolymeren davon und Salzen davon, dem anionischen verzweigten Block-Butylacrylat/Acrylsäure/Methacrylsäure-Polymer (INCI-Name: AMP-Acrylate/Allylmethacrylat-Copolymer), anionischen Polysacchariden, allein oder als Mischungen.

2. Zusammensetzung nach einem der vorhergehenden Ansprüche, **dadurch gekennzeichnet, dass** das Polymer (a) ein C₁-C₄-Alkyl(meth)acrylat-Polymer ist.

3. Zusammensetzung nach einem der vorhergehenden Ansprüche, **dadurch gekennzeichnet, dass** das Polymer (a) ein ethylenisch ungesättigtes Säuremonomer oder dessen Anhydrid umfasst, vorzugsweise ausgewählt aus (Meth)acrylsäure, Maleinsäure und Maleinsäureanhydrid.

4. Zusammensetzung nach einem der vorstehenden Ansprüche, **dadurch gekennzeichnet, dass** das Polymer (a) ausgewählt ist aus:
- Methylacrylat-Homopolymeren,
- Ethylacrylat-Homopolymeren,
- Methylacrylat/Ethylacrylat-Copolymeren,
- Methylacrylat/Ethylacrylat/Acrylsäure-Copolymeren,
- Methylacrylat/Ethylacrylat/Maleinsäureanhydrid-Copolymeren,
- Methylacrylat/Acrylsäure-Copolymeren,
- Ethylacrylat/Acrylsäure-Copolymeren,
- Methylacrylat/Maleinsäureanhydrid-Copolymeren,
- Ethylacrylat/Maleinsäureanhydrid-Copolymeren.

5. Zusammensetzung nach einem der vorhergehenden Ansprüche, **dadurch gekennzeichnet, dass** das nichtwässrige Medium mindestens ein Öl umfasst, das aus Ölen auf Kohlenstoffbasis, Ölen auf Kohlenwasserstoffbasis, Fluorölen und/oder Silikonölen mineralischen, tierischen, pflanzlichen oder synthetischen Ursprungs und Mischungen davon ausgewählt ist.

6. Zusammensetzung nach einem der vorhergehenden Ansprüche, **dadurch gekennzeichnet, dass** das nichtwässrige Medium mindestens ein Öl auf Kohlenwasserstoffbasis umfasst, vorzugsweise ausgewählt aus:
(i) Ölen auf Kohlenwasserstoffbasis mit 8 bis 16 Kohlenstoffatomen;
(ii) kurzkettigen Estern mit insgesamt 3 bis 8 Kohlenstoffatomen;
(iii) Ölen auf Kohlenwasserstoffbasis pflanzlichen Ursprungs;
(iv) synthetischen Ethern mit 10 bis 40 Kohlenstoffatomen;
(v) linearen oder verzweigten Kohlenwasserstoffen mineralischen oder synthetischen Ursprungs;
(vi) synthetischen Estern;
(vii) bei Raumtemperatur flüssigen Fettalkoholen mit einer verzweigten und/oder ungesättigten Kette auf Kohlenstoffbasis mit 12 bis 26 Kohlenstoffatomen; und
(viii) Mischungen davon.

7. Zusammensetzung nach einem der vorhergehenden Ansprüche, **dadurch gekennzeichnet, dass** der Stabilisator ein Isobornyl(meth)acrylat-Polymer ist, ausgewählt aus Isobornyl(meth)acrylat-Homopolymer und statistischen Copolymeren von Isobornyl(meth)acrylat und von C₁-C₄-Alkyl(meth)acrylat, die bevorzugt bei einem Isobornyl(meth)acrylat/C₁-C₄-Alkyl(meth)acrylat-Gewichtsverhältnis größer als 4, stärker bevorzugt größer als 4,5 und noch bevorzugter größer als oder gleich 5 vorhanden sind.

8. Zusammensetzung nach einem der vorhergehenden Ansprüche, **dadurch gekennzeichnet, dass** der Gesamtgehalt an Partikeldispersion(en) im Bereich von 0,1 bis 20 Gew.-%, stärker bevorzugt von 0,5 bis 10 Gew.-% und noch bevorzugter von 1 bis 8 Gew.-%, bezogen auf das Gewicht der Zusammensetzung, liegt.

9. Zusammensetzung nach einem der vorhergehenden Ansprüche, **dadurch gekennzeichnet, dass** die kationischen Polymere aus kationischen Polysacchariden ausgewählt sind.

10. Zusammensetzung nach einem der vorhergehenden Ansprüche, **dadurch gekennzeichnet, dass** der Gehalt des kationischen Polymers bzw. der kationischen Polymere im Bereich von 0,05 bis 15 Gew.-%, vorzugsweise von 0,5 bis 10 Gew.-% und stärker bevorzugt von 1 bis 5 Gew.-%, bezogen auf das Gesamtgewicht der Zusammensetzung, liegt.

11. Zusammensetzung nach einem der vorhergehenden Ansprüche, **dadurch gekennzeichnet, dass** der Gehalt des anionischen Polymers bzw. der anionischen Polymere im Bereich von 0,05 bis 15 Gew.-%, stärker bevorzugt von 0,1 bis 10 Gew.-% und noch bevorzugter von 0,5 bis 5 Gew.-%, bezogen auf das Gesamtgewicht der Zusammensetzung, liegt.

12. Zusammensetzung nach einem der vorhergehenden Ansprüche, **dadurch gekennzeichnet, dass** das Gewichtsverhältnis von kationischem(n) Polymer(en)/anionischem(n) Polymer(en) im Bereich von 10/90 bis 90/10, besser noch von 20/80 bis 80/20 liegt; wobei dieses Gewichtsverhältnis bevorzugt im Bereich von 30/70 bis 70/30 und noch bevorzugter von 40/60 bis 60/40 liegt.

13. Zusammensetzung nach einem der vorhergehenden Ansprüche, **dadurch gekennzeichnet, dass** die Zusammensetzung Wasser in einem Gehalt im Bereich von 30 bis 98 Gew.-%, vorzugsweise von 50 bis 98 Gew.-% und stärker bevorzugt von 65 bis 95 Gew.-%, bezogen auf das Gesamtgewicht der Zusammensetzung, umfasst.

14. Kosmetisches Verfahren zur Behandlung von Keratinfasern, insbesondere menschlichen Keratinfasern, wie z. B. dem Haar, umfassend das Aufbringen einer Zusammensetzung nach einem der vorstehenden Ansprüche auf die Keratinfasern.

15. Verwendung einer Zusammensetzung nach einem der Ansprüche 1 bis 13 zum Styling von Keratinfasern, insbesondere menschlichen Keratinfasern, wie z. B. dem Haar.

## Revendications

1. Composition comprenant :
(a) une ou plusieurs dispersions de particules d'au moins un polymère, stabilisées par un ou plusieurs stabilisants polymères dans un milieu non aqueux,
(b) un ou plusieurs polymères cationiques et
(c) un ou plusieurs polymères anioniques, dans laquelle :
- le stabilisant est un polymère de (méth)acrylate d'isobornyle,
- le polymère (a) sous la forme de particules est un polymère éthylénique,
- les polymères cationiques sont choisis parmi les homopolymères ou copolymères renfermant une ou plusieurs unités dérivées de vinylamine et éventuellement une ou plusieurs unités dérivées de vinylformamide, les polysaccharides cationiques, les cyclopolymères d'alkyldiallylamine ou de dialkyldiallylammonium et leurs mélanges,
- les polymères anioniques sont choisis parmi les homopolymères ou copolymères d'acide acrylique ou méthacrylique réticulés ou non réticulés et les sels de ceux-ci, les homopolymères d'acide 2-acrylamido-2-méthylpropanesulfonique réticulés ou non réticulés et les sels de ceux-ci et les copolymères d'acrylamide de ceux-ci réticulés ou non réticulés et les sels de ceux-ci, le polymère anionique à blocs ramifié d'acrylate de butyle/acide acrylique/acide méthacrylique (nom INCI : AMP-acrylates/allyl méthacrylate copolymer), les polysaccharides anioniques, seuls ou sous forme de mélanges.

2. Composition selon l'une ou l'autre des revendications précédentes, **caractérisée en ce que** le polymère (a) est un polymère de (méth)acrylate d'alkyle en C₁-C₄.

3. Composition selon l'une quelconque des revendications précédentes, **caractérisée en ce que** le polymère (a) comprend un monomère acide à insaturation éthylénique ou l'anhydride de celui-ci, de préférence choisi parmi l'acide (méth)acrylique, l'acide maléique et l'anhydride maléique.

4. Composition selon l'une quelconque des revendications précédentes, **caractérisée en ce que** le polymère (a) est choisi parmi :
- les homopolymères d'acrylate de méthyle,
- les homopolymères d'acrylate d'éthyle,
- les copolymères d'acrylate de méthyle/acrylate d'éthyle,
- les copolymères d'acrylate de méthyle/acrylate d'éthyle/acide acrylique,
- les copolymères d'acrylate de méthyle/acrylate d'éthyle/anhydride maléique,
- les copolymères d'acrylate de méthyle/acide acrylique,
- les copolymères d'acrylate d'éthyle/acide acrylique,
- les copolymères d'acrylate de méthyle/anhydride maléique,
- les copolymères d'acrylate d'éthyle/anhydride maléique.

5. Composition selon l'une quelconque des revendications précédentes, **caractérisée en ce que** le milieu non aqueux comprend au moins une huile choisie parmi les huiles à base de carbone, les huiles à base d'hydrocarbures, les huiles fluorées et/ou les huiles de silicone d'origine minérale, animale, végétale ou synthétique et les mélanges de celles-ci.

6. Composition selon l'une quelconque des revendications précédentes, **caractérisée en ce que** le milieu non aqueux comprend au moins une huile à base d'hydrocarbures, de préférence choisie parmi :
(i) les huiles à base d'hydrocarbures contenant de 8 à 16 atomes de carbone ;
(ii) les esters à chaîne courte contenant de 3 à 8 atomes de carbone au total ;
(iii) les huiles à base d'hydrocarbures d'origine végétale ;
(iv) les éthers synthétiques contenant de 10 à 40 atomes de carbone ;
(v) les hydrocarbures linéaires ou ramifiés, d'origine minérale ou synthétique ;
(vi) les esters synthétiques ;
(vii) les alcools gras qui sont liquides à température ambiante, à chaîne à base de carbone ramifiée et/ou insaturée contenant de 12 à 26 atomes de carbone ; et
(viii) les mélanges de ceux-ci.

7. Composition selon l'une quelconque des revendications précédentes, **caractérisée en ce que** le stabilisant est un polymère de (méth)acrylate d'isobornyle choisi parmi un homopolymère de (méth)acrylate d'isobornyle et les copolymères statistiques de (méth)acrylate d'isobornyle et de (méth)acrylate d'alkyle en C₁-C₄ de préférence présent en un rapport pondéral (méth)acrylate d'isobornyle/(méth)acrylate d'alkyle en C₁-C₄ supérieur à 4, plus préférentiellement supérieur à 4,5 et mieux encore supérieur ou égal à 5.

8. Composition selon l'une quelconque des revendications précédentes, **caractérisée en ce que** la teneur totale de la ou des dispersions de particules se situe dans la plage allant de 0,1 % à 20 % en poids, plus préférentiellement de 0,5 % à 10 % en poids et mieux encore de 1 % à 8 % en poids par rapport au poids de la composition.

9. Composition selon l'une quelconque des revendications précédentes, **caractérisée en ce que** les polymères cationiques sont choisis parmi les polysaccharides cationiques.

10. Composition selon l'une quelconque des revendications précédentes, **caractérisée en ce que** la teneur du ou des polymères cationiques se situe dans la plage allant de 0,05 % à 15 % en poids, de préférence de 0,5 % à 10 % en poids et plus préférentiellement de 1 % à 5 % en poids, par rapport au poids total de la composition.

11. Composition selon l'une quelconque des revendications précédentes, **caractérisée en ce que** la teneur du ou des polymères anioniques se situe dans la plage allant de 0,05 % à 15 % en poids, plus préférentiellement de 0,1 % à 10 % en poids et mieux encore de 0,5 % à 5 % en poids, par rapport au poids total de la composition.

12. Composition selon l'une quelconque des revendications précédentes, **caractérisée en ce que** le rapport pondéral polymère(s) cationique(s)/polymère(s) anionique(s) se situe dans la plage allant de 10/90 à 90/10, mieux encore de 20/80 à 80/20 ; de préférence, ce rapport pondéral se situe dans la plage allant de 30/70 à 70/30 et plus préférentiellement de 40/60 à 60/40.

13. Composition selon l'une quelconque des revendications précédentes, **caractérisée en ce que** la composition comprend de l'eau en une teneur se situant dans la plage allant de 30 % à 98 % en poids, de préférence de 50 % à 98 % en poids et plus préférentiellement de 65 % à 95 % en poids, par rapport au poids total de la composition.

14. Procédé cosmétique pour le traitement de fibres de kératine, en particulier de fibres de kératine humaines telles que les cheveux, comprenant l'application sur lesdites fibres de kératine d'une composition telle que définie dans l'une quelconque des revendications précédentes.

15. Utilisation d'une composition telle que définie dans l'une quelconque des revendications 1 à 13, pour le coiffage de fibres de kératine, en particulier de fibres de kératine humaines telles que les cheveux.
